# EUROPEAN PATENT SPECIFICATION

(11) **EP 2 519 350 B1**
(45) Date of publication and mention of the grant of the patent: **20.03.2019**
(21) Application number: 10840586.1
(22) Date of filing: 30.12.2010
(51) Int. Cl.: B01J 31/22, C07F 15/00, C08F 4/80, C08C 19/08

(54) **HIGHLY ACTIVE METATHESIS CATALYSIS SELECTIVE FOR ROMP AND RCM**
FÜR ROMP UND RCM SELEKTIVE HOCHAKTIVE METATHESEKATALYSATOREN
CATALYSEUR DE MÉTATHÈSE FORTEMENT ACTIF SÉLECTIF VIS-À-VIS DE ROMP ET RCM

(30) Priority: 30.12.2009 WO PCT/CN2009/076226
(43) Date of publication of application: 07.11.2012
(60) Divisional application: 16164005.7
(73) Proprietor: Zannan Scitech Co., Ltd., Shangai 201 108 (CN)
(72) Inventor: ZHAN, Zheng-Yun James, Shanghai 201108 (CN)
(74) Representative: Biggi, Cristina
(86) International application number: PCT/CN2010/080477
(87) International publication number: WO 2011/079799

(56) References cited:
- WO-A1-2005/016944
- WO-A1-2005/080456
- WO-A1-2007/003135
- WO-A1-2009/124853
- WO-A2-2010/096445
- CN-A- 101 684 075
- US-A1- 2004 132 891
- TANIYAMA D ET AL: "A facile asymmetric synthesis of 1-substituted tetrahydroisoquinoline based on a chiral ligand-mediated addition of organolithium to imine", TETRAHEDRON ASYMMETRY, PERGAMON PRESS LTD, OXFORD, GB, vol. 10, no. 2, 29 January 1999 (1999-01-29), pages 221-223, XP004158841, ISSN: 0957-4166, DOI: 10.1016/S0957-4166(98)00509-6
- SLUGOVC, CHRISTIAN ET AL.: 'Thermally Switchable Olefin Metathesis Initiators Bearing Chelating Carbenes: Influence of the Chelate's Ring Size.' ORGANOMETALLICS vol. 24, no. 10, 08 April 2005, pages 2255 - 2258, XP002455276
- EVANS, PAUL ET AL.: 'Synthesis of a 6-aryloxymethyl-5-hydroxy-2,3,4,5-tetrahydr o-[lH]-2- enzazepin-4-one: a muscarinic (M3) antagonist.' ORGANIC & BIOMOLECULAR CHEMISTRY vol. 6, 2008, pages 2158 - 2167, XP008155287
- POGOSYAN, G. M. ET AL.: 'Styrene derivatives. XXII. Synthesis and polymerization of amines of 2-vinylbenzoic acid.' ARMYANSKII KHIMICHESKII ZHURNAL vol. 24, no. 9, 1971, pages 816 - 821, XP008155291
- SUTTHASUPA, SUTTHIRA ET AL.: 'ROMP of Norbornene Monomers Carrying Nonprotecte Amino Groups with Ruthenium Catalyst.' MACROMOLECULES vol. 42, no. 5, 12 February 2009, pages 1519 - 1525, XP008155298
- BERTIN, PAUL A. ET AL.: 'High-density doxorubicin-conjugated polymeric nanoparticles via ring-opening metathesis polymerization.' CHEM. COMMUN. 2005, pages 3793 - 3795, XP008117650
- VYGODSKII, YAKOV S. ET AL.: 'Ring-Opening Metathesis Polymerization (ROMP) in Ionic Liquids: Scope and Limitations.' MACROMOLECULES vol. 39, no. 23, 20 October 2006, pages 7821 - 7830, XP008155300

## Description

### FIELD OF THE INVENTION

The present invention relates to novel carbene ligands and their incorporated ruthenium catalysts, which are highly active and selective for different kinds of metathesis reactions such as ROMP and RCM. The invention also relates to preparation of new ruthenium complexes and the use thereof in metathesis, especially effective for preparation of various functional polymers and rubbers.

### BACKGROUND OF THE INVENTION

Since Richard R. Schrock and Robert H. Grubbs prepared two kinds of metathesis catalysts with transition metal carbene structure in the 1990's, it has been drawning extensive attention in the development of more active and selective ruthenium catalysts for different kinds of olefin metathesis reactions, e.g., ring-opening metathesis polymerization (ROMP), ring-closing metathesis (RCM), and cross metathesis (CM).

So far, some useful ruthenium complexes have been reported as active metathesis catalysts (**1a-1b** and **2a-2f** in Scheme 1) for RCM and ROMP reactions (Grubbs et al., J. Am. Chem. Soc. 1992, 114, 3974-3975, Org. Lett. 1999, 1, 953-956, WO2007081987A1; Hoveyda et al., J. Am. Chem. Soc. 1999, 121, 791-799, J. Am. Chem. Soc. 2000, 122, 8168-8179; Yamaguchi et al., Chem. Commun. 1998, 1399-1400; Zhan et al., US20070043180A1, WO 2007003135A1; Grela et al., WO2004035596A1; Slugovc et al., Organometallics 2004, 23(15), 3623-3626 for catalyst **2d**; and Organometallics 2005, 24(10), 2255-2258 for catalyst **2e**). However, a disadvantage of all reported ruthenium catalysts is obviously substrate-dependent for different kinds of ruthenium catalysts in metathesis reactions, and it is still very difficult to find some active metathesis catalysts selective for RCM and ROMP reactions, respectively. Moreover, only a few metathesis catalysts could be used effectively to make high-strength and high-stiffness polydicyclopentadiene (PDCPD) material by ROMP reaction.

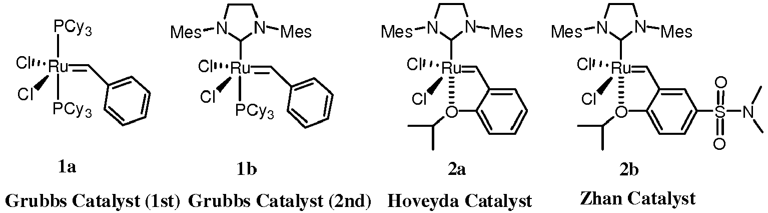

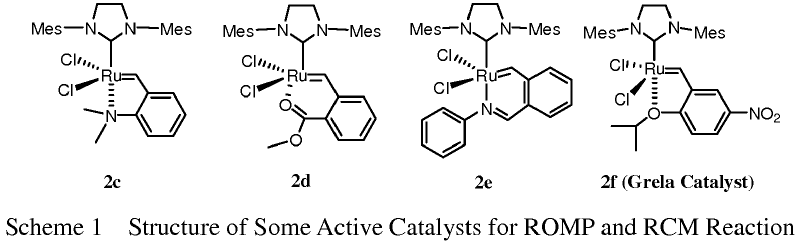

Currently, ROMP reaction is broadly used for preparation of various high-strength and other functional polymers. To overcome the activity and selectivity problems for ROMP catalysts, it has become a goal to develop more active and selective metathesis catalysts as an alternative for ROMP and RCM reactions, especially in ROMP for effective preparation and modification of different functional polymer materials. It is significantly important to develop more active and selective ruthenium catalyst for ROMP reactions with different kinds of olefin substrates to prepare highly functional polymer materials and also to improve polymer properties.

WO2010/096445A2 discloses the ROMP of cyclic olefins in the presence of ruthenium complexes in which one ruthenium is bonded to four or five ligands. US2004/0132891 discloses a process for the production of hydrogenated nitrile rubber polymers, wherein the lower Mooney viscosity of nitrile rubber polymers are prepared by using the 2^{nd} generation of Grubbs Catalyst in which a transition metal ruthenium is bonded to various ligands to form five bonds. Neither of these two patent applications discloses the structures of the ruthenium complexes with various new ligands to form six bonds claimed by the present application.

### SUMMARY OF THE INVENTION

The present invention relates to two classes of novel carbene ligands and their incorporated ruthenium complexes that can be used as highly active metathesis catalysts selective for RCM, CM, and ROMP reactions, respectively. The novel metathesis catalysts are ruthenium complexes with different kinds of new functionally substituted carbene ligands. The new ruthenium complexes of the invention can catalyze different kinds of metathesis reactions in a very effective manner and offer great advantage in activity and selectivity for different kinds of metathesis reactions, especially in ROMP effective for preparation of some functional polymer materials with unique chemical and physical properties. The novel ruthenium complexes of the invention have broad uses in the polymeric and pharmaceutical industries.

In the first aspect, the present invention provides a kind of transition metal complex having the following structure **IIa:** 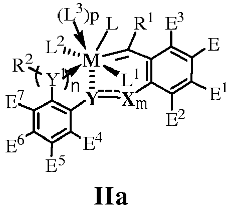 wherein:
m = 0 or 1, n = 1;
M is ruthenium;
L¹ and L² each is chloride anion;
L is an electron-donating ligand having the following structure **IIIa** or **IIId:** 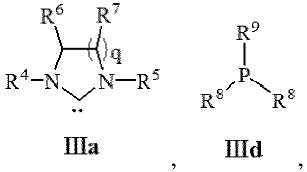
and in **IIIa**, q = 1, R⁴ and R⁵ each is 2,4,6-trimethylphenyl, R⁶ and R⁷ each is H; or in **IIId**, R⁸ and R⁹ each is cyclohexyl (Cy);
when m = 1, X is CH₂; Y is NH, or C₁-C₄ alkylamino; "**Y⁻⁻⁻X**" is single bond;
when m = 0, Y is NH, C₁-C₄ alkylamino,or C₆-C₉ arylamino;
n = 1, p = 0;
Y¹ is oxygen;
R¹ is H;
R² is methyl, ethyl, or isopropyl;
E is H, halogen, nitro, C₁-C₄ alkoxy, C₁-C₄ alkoxycarbonyl, C₁-C₈ alkylaminosulfonyl;
E¹ and E² are each H or halogen;
E³ is H;
E⁴ is H or C₁-C₄ alkyl;
E⁵ and E⁶ is H, halogen, C₁-C₄ alkyl or C₁-C₆ alkoxy;
E⁷ is H or C₁-C₄ alkyl.

In the second aspect, the present invention provides the following synthetic methods of making different kinds of transition metal complexes **IIa**.

First of all, in the present invention, when Z is CH₂, the complex ligands **Ia** could be prepared by the following Suzuki reaction: 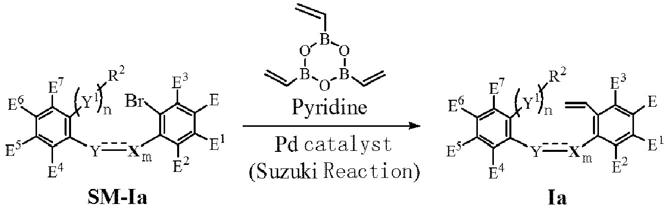 Wherein Y, Y¹, R¹, R², E, E¹, E² and E³ each is as defined above.

The ligands **Ia** can be prepared by the coupling of chemicals SM-1a or SM-Ib with vinyl borane reagent in organic solvent such as DMF in the prence of Pd catalyst. SM-1a were ordered by custom synthesis from Zannan Pharma Ltd, in China.

Method **1** in the following Scheme 1:

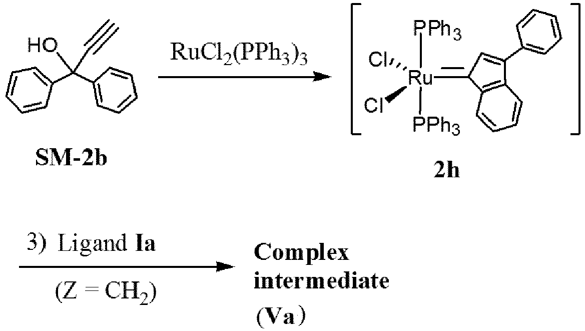

The intermediate of transition metal complex (**Va**) having the following structure: 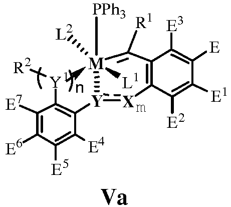
(1) The Ru complex **2h** is prepared by the reaction of reagent **SM-2b** and RuCl₂(PPh₃)₃ in anhydrous DCM in a three-neck flask filled with inert gas (Ar).
(2) The Ru complex **2h** obtained by step (1) is reacted with complex ligand **Ia** to prepare another Ru complex **Va** in a flask filled with inert gas (Ar); wherein, **Va** is compound **IIa** when L is PPh₃; M, L¹, L², Y, Y¹, R¹, R², E, E¹, E² and E³ are each as defined above.

More preferred, L¹ and L² each is chloride anion (Cl⁻).

Wherein, in step (1), the preferred one of E and X¹ is hydrogen; the preferred usage of anhydrous organic solvent is 5-30 times weight of **SM-2**; the more preferred usage is 15 times; the preferred reaction temperature is 25-75°C, the more preferred temperature is 50-65 °C.

In step (2), the preferred reaction temperature is -50°C to -85°C, the more preferred temperature is -60°C to -75°C; the preferred usage of ML¹L²L₃ is 0.3-1.0 times molar ratio of **SM-2**, the more preferred usage is 0.6-0.7 times; the preferred compound of ML¹L²L₃ is RuCl₂(PPh₃)₃;

In step (3) of method 1, the preferred reaction temperature is -50°C to -85°C, the more preferred temperature is -60°C to -75°C; the preferred usage of complex ligand **Ia** or **Ib** is 1-3 molar ratio of complex intermediate, the preferred usage is 1.5-2 eq. When ML¹L²L is RuCl₂(PPh₃)₃, the structure of product **Va** is as follows:

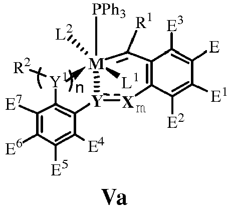

Method **2:** the complex **Va** obtained by method 1 is reacted respectively with any electron-donating complex ligand L except PPh₃ to prepare following metal complexes **IIa**, wherein, p = 0, q = 1, definition of M, L, L¹, L², Y, Y¹, R¹, R², E, E¹, E² and E³ each is as defined above;

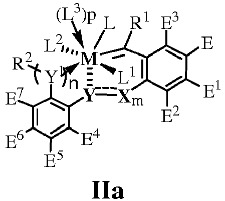

Wherein, the preferred one, in structure of transition metal complexs as the product **IIa**, where a preferred ligand L is **IIIa** or **IIId**. The preferred reaction termperature is 20°C to 75°C, the more preferred reaction temperature reacted with complex ligand **IIIa** is 60°C to 75°C, the more preferred reaction temperature reacted with complex ligand IIId is 20°C to 35°C; The preferred usage of **IIIa** or **IIId** is 1-3 times molar ratio of complex intermediate **Va**, the more preferred molar ratio is 1.5-2 eq;

Method **3:** when L is PCy₃ or PPh₃, the **IIa** is reacted respectively with any electron-donating complex ligand L (**IIIa**) or L³ to prepare the metal complex **IIa**, wherein p = 0, M, L¹, L², Y, Y¹, R¹, R², E, E¹, E², E³ is each as defined above.

Method **4:** when L is PCy₃ or **IIIa**, the **IIa** is reacted respectively with any electron-donating complex ligand L³ to prepare the metal complex **IIa**, wherein p = 1, M, L¹, L², Y, Y¹, R¹, R², E, E¹, E², E³ is each as defined above. In method **4**, the preferred reaction temperature is 20°C to 35°C.

From method **1** to method **4**, L¹ and L² each is chloride anion.

Based on currently developed technology, the metal complex **IIa** of the present invention could also be prepared by the following two alternative procedures described in Schemes 2 & 3:

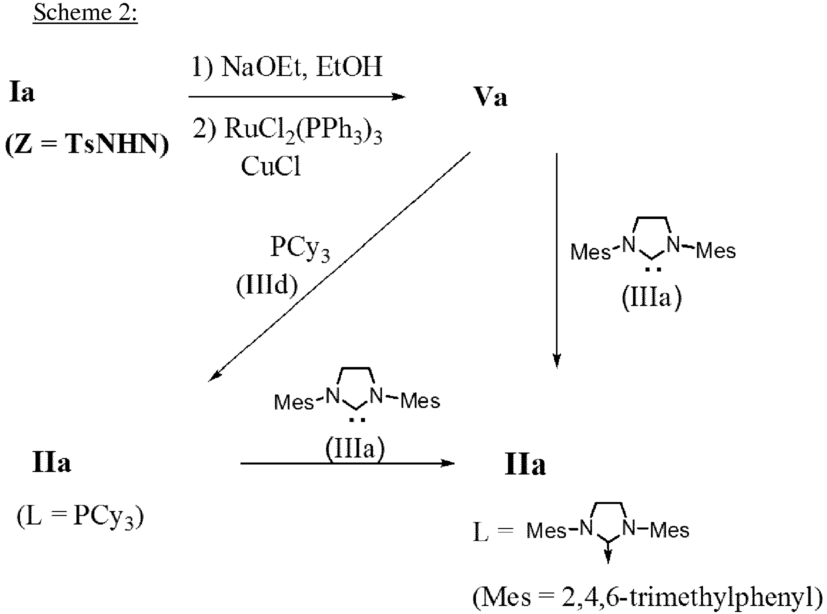

In the above procedure, Z is TsNHN in structure **Ia**.

In Scheme 2, **Ia** reacts with NaOEt in anhydrous EtOH to form carbene in a flask filled with inert gas, followed by reacting with RuCl₂P(Ph₃)₃ to form complex **Va**. The complex **Va** is reacted with **IIIa** or **IIId** effectively to obtain the complex **IIa** in inert gas, respectively.

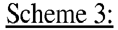

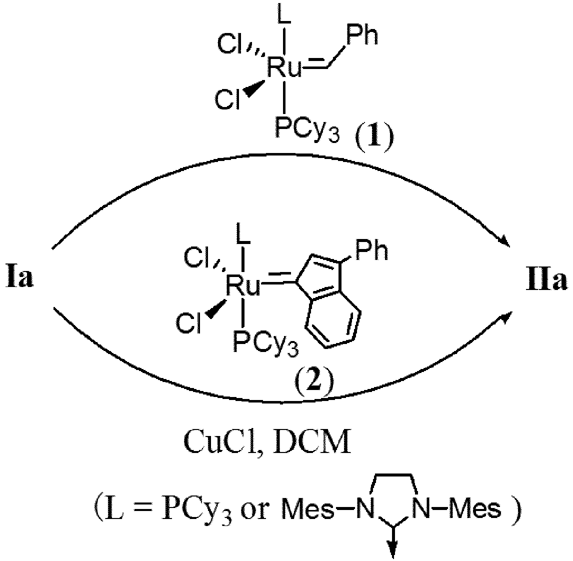

Complexes **IIa** could also be prepared by other two alternative synthetic routes as shown in Scheme 3.

In Scheme 3, **Ia** reacts with ruthenium complex **1** or **2** directly to form the desired complex **IIa** in a flask filled with inert gas (Ar), respectively.

In the second aspect, the present invention provides a method of carrying out a metathesis reaction effectively with olefin substrate, comprising intramolecular ring-closing metathesis (RCM), intermolecular cross metathesis (CM), acyclic diene metathesis (ADMET), or ring-opening metathesis polymerization (ROMP) of cyclo-olefin substrate selectively in the presence of the novel ruthenium catalysts.

In the third preferred embodiment, the present invention provides a method of making a modified nitrile butadiene rubber (NBR) or styrene-butadiene rubber (SBR) by depolymerization in the presence of one or more mixed catalysts of the present invention at 30-100°C.

In the fourth preferred embodiment, the present invention provides a method of making a depolymerized HNBR (hydrogenated nitrile butadiene rubber) or styrene-butadiene rubber by adding one or more mixed catalysts of the present invention first to carry out depolymerization of NBR, followed by adding hydrogen into the reaction under high pressure for hydrogenation at 60-150°C.

In the fifth preferred embodiment, the present invention provides a method of making a hydrogenated nitrile butadiene rubber (HNBR) or styrene-butadiene rubber by adding hydrogen under high pressure first, followed by adding one or more mixed catalysts of claim 5 at 60-150°C.

In the sixth preferred embodiment, the present invention provides a use of catalysts of the present invention in depolymerization of a rubber comprising at least one carbon-carbon double bond.

In the seventh preferred embodiment, the present invention provides a use of catalysts of the present invention in hydrogenation of a rubber comprising at least one carbon-carbon double bond.

The present invention relates to one class of novel carbene ligands and ruthenium complexes as catalysts in uses of carrying out metathesis reactions effectively, e.g., preparation of the high strength and/or stiffness polymer materials, functional polymers linked with small molecule pro-drugs and liquid crystal materials.

Currently, the present invention provides the following significant achievements:
1. One class of novel carbene ligands and ruthenium complexes have been designed and prepared, which has different structure and activity with five or six coordinate bonds, especially for the new Ru complexes with six coordinate bonds to form at least one "Ru-N" coordinate bond from the new developed ligands **Ia**. Moreover, the electronic and steric effect of different substituted ligands on the catalytic activity and stability of various new Ru complexes have been studied, and it is found that some of novel Ru catalysts in the present invention have much better catalytic selectivity and variable physical diversity than Grubbs and Hoveyda catalysts in the ROMP and RCM reactions.
2. The experimental results show that some of novel Ru catalysts in the present invention have high activity and selectivity for different olefin ROMP and RCM reactions, so the invention provides a useful synthetic method of carrying out olefin metathesis reactions effectively in preparation of polymer materials and pharmaceutical intermediates.
3. The present invention provides several developed methods for preparation of carbene ligands and Ru catalysts at lower cost, and it also provides some efficient methods for preparation of various functional polymer materials with different chemical and physical properties.
4. The present invention provides several developed processes of conducting ROMP reaction with one or two more mixed of novel active Ru catalysts for preparation of the high-strength polymer materials and some functional polymers linked with small molecule pro-drugs and/or liquid crystal materials.

### BRIEF DESCRIPTION OF THE DRAWINGS

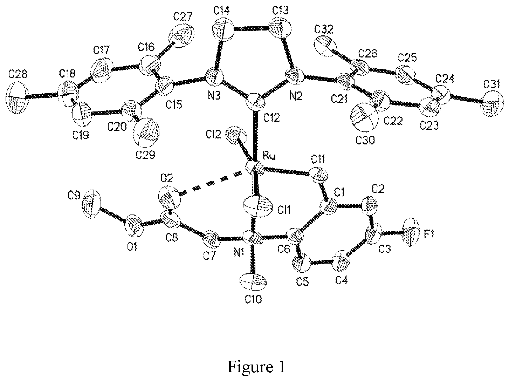

Figure 1: Single-crystal X-ray Structure of Ru Catalyst **8m. (Not a part of the present application)**

### DETAILED DESCRIPTION OF THE INVENTION

The present invention comprises two novel classes of carbene ligands and ruthenium complexes as catalysts for metathesis reactions. To study the electronic and steric effects of multi-substituted benzylidene ligands on the stability and activity of Ru complexes, based on the following reported procedure in Schemes 1-3, different kinds of the complex ligands (**3a-3bf**) have been prepared and reacted with the Ru complex **1** to obtain different kinds of new Ru complex (**4a-4bf**). During preparation and activity evaluation of various Ru complexes with various substituted 2-aminobenzylidene ligands in the following Schemes 4-8, different kinds of the electron-withdrawing and/or electron-donating effect and steric effect on the stability and selective activity for ROMP and RCM reactions have been found as shown in Schemes 9-16 and Tables 1-6.

**Significant electronic effect of various substituted benzylidene ligands on the stability of Ru complexes:** Based on different described synthetic methods and procedures in Schemes 1-3, there are different kinds of new olefin or carbene ligands (**Ia**) and Ru complexes (**IIa**) prepared in the present invention. Moreover, significant substituent effect of different substituted benzylidene ligands on the stability and activity of Ru complexes has been observed and developed selectively for ROMP and RCM reactions, and some novel Ru catalysts have been prepared much more active and selective than prior reported Ru catalysts for different kinds of ROMP and RCM reactions.

According to previously described synthetic methods, various new Ru complexes **4a-4bf** have been prepared by the reaction listed in Scheme 4, and the corresponding metathesis activity of each Ru complex has been studied for RCM and ROMP reactions with different olefin substrates, respectively.

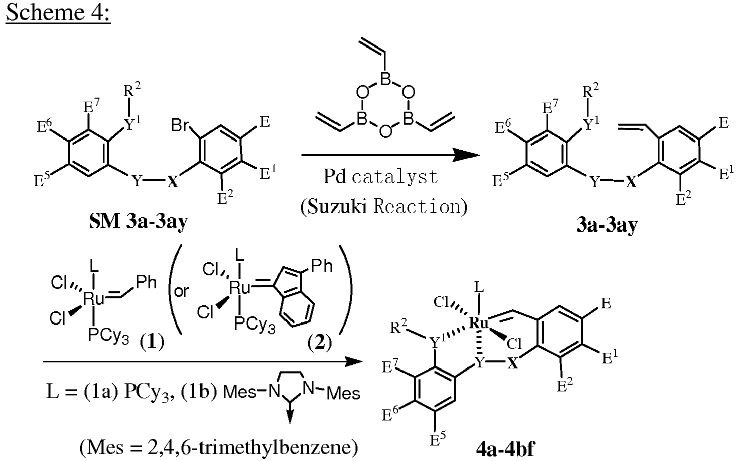

Some selected structure of prepared ligands **3a-3bf** and corresponding ruthenium complexes **4a-4bf** (**Ia**: Cy = cyclohexyl, **1b** = 2,4,6-trimethylbenzene) are listed as follows:

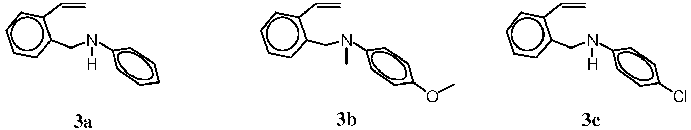

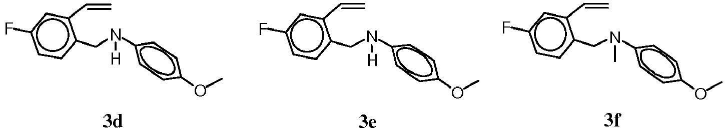

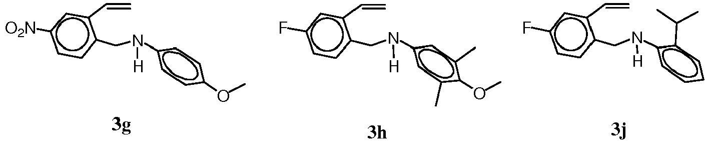

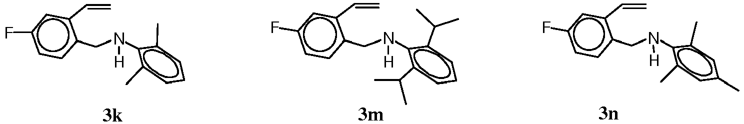

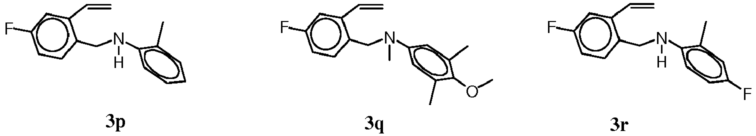

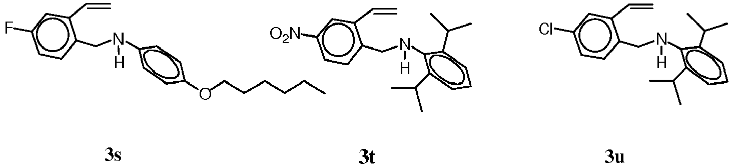

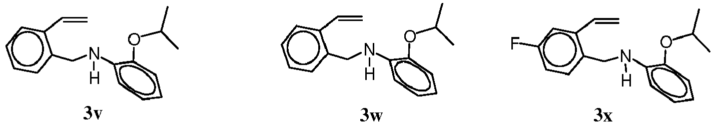

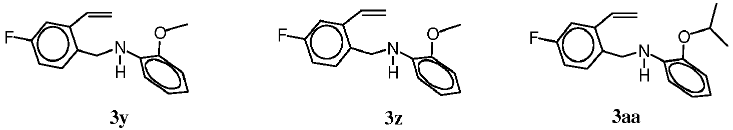

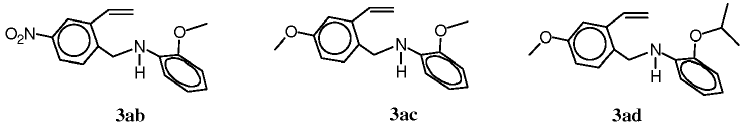

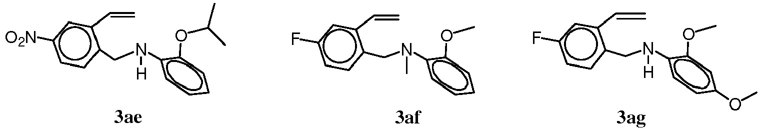

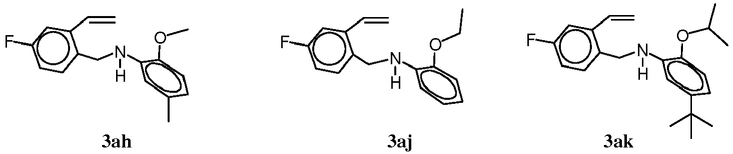

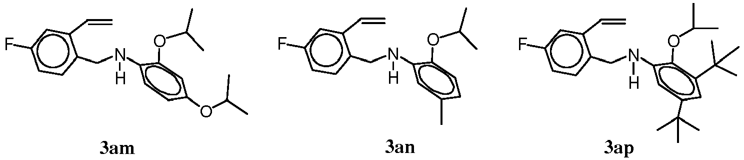

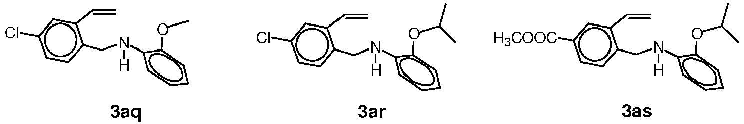

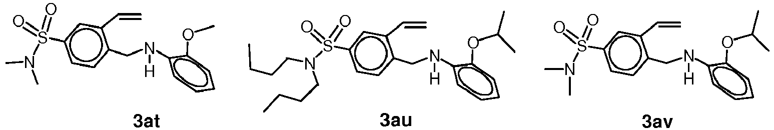

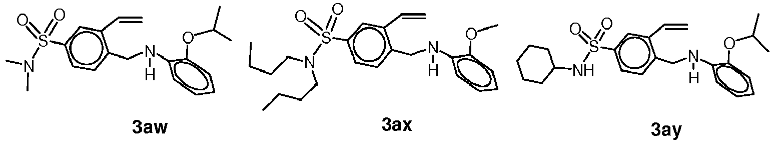

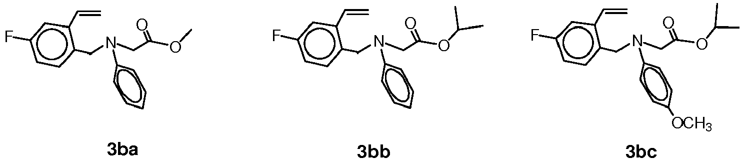

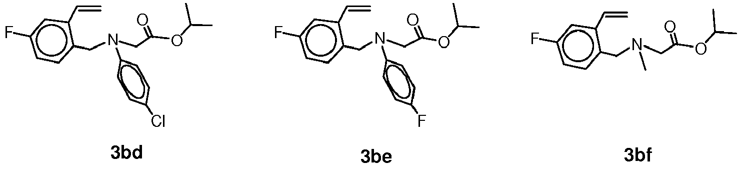

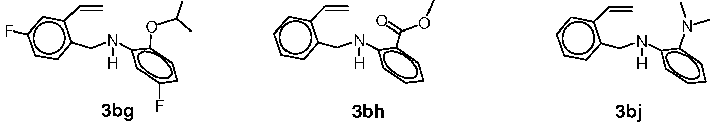

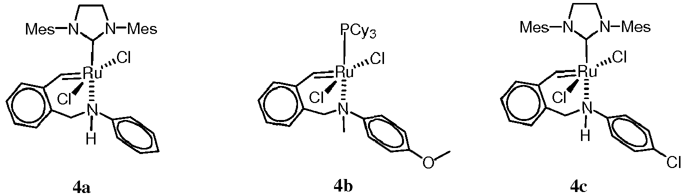

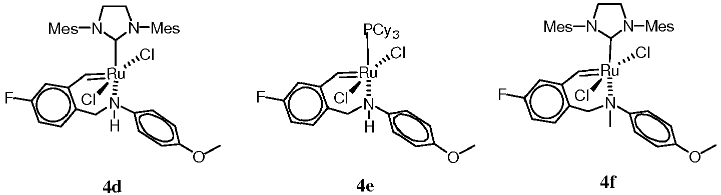

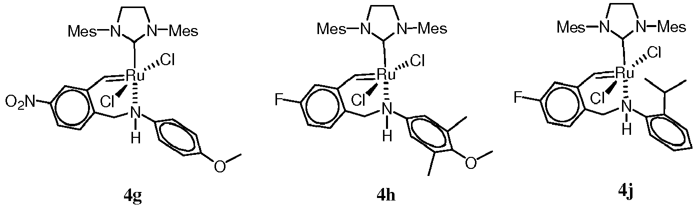

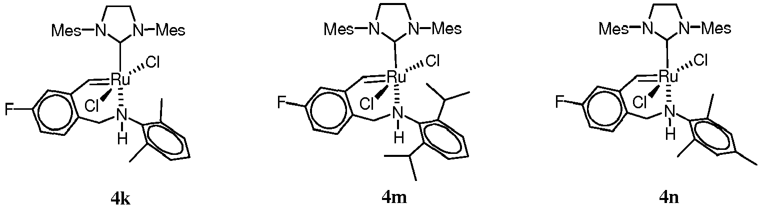

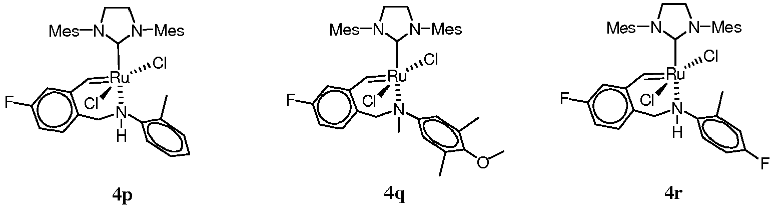

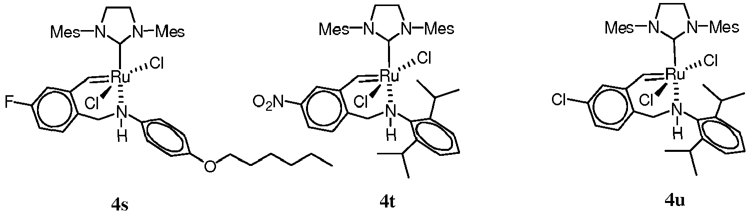

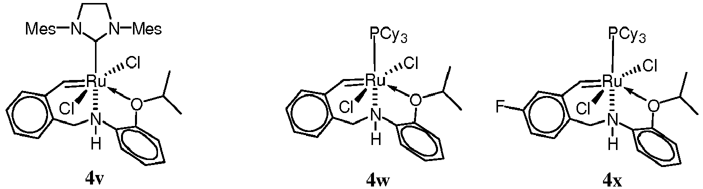

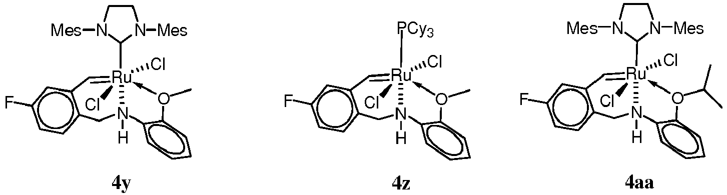

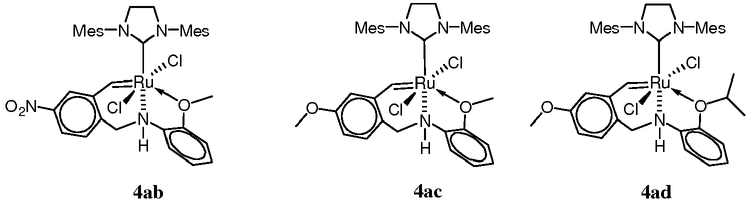

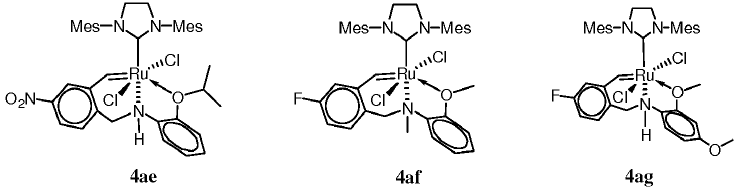

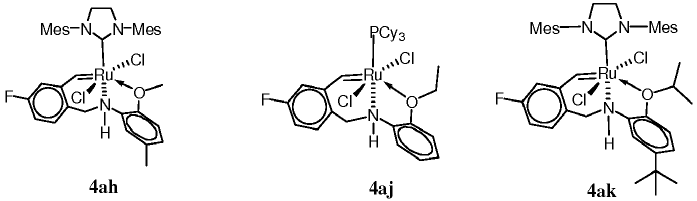

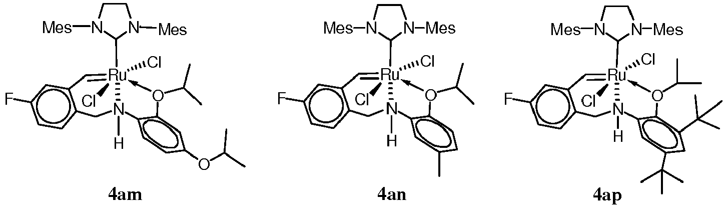

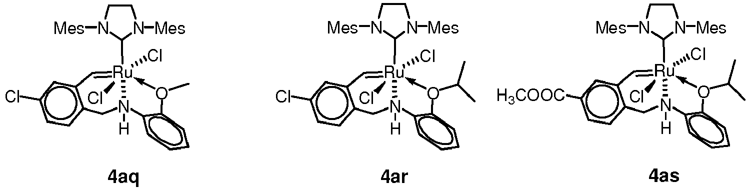

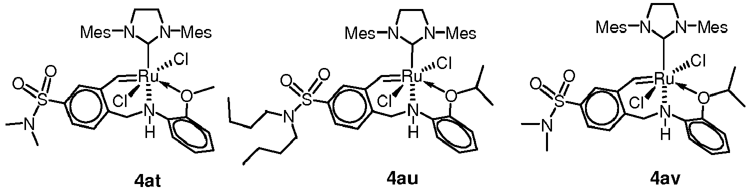

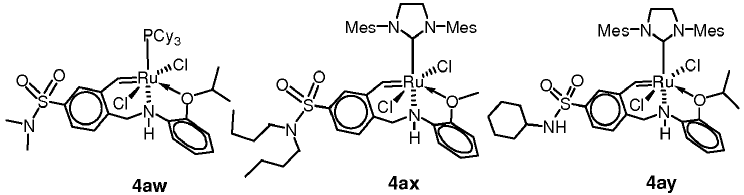

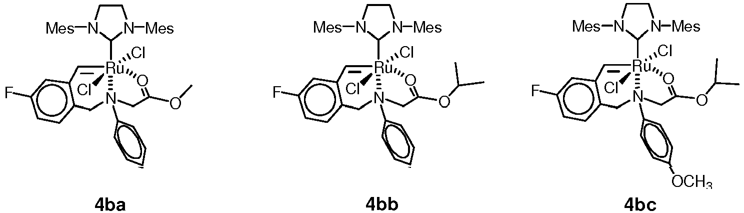

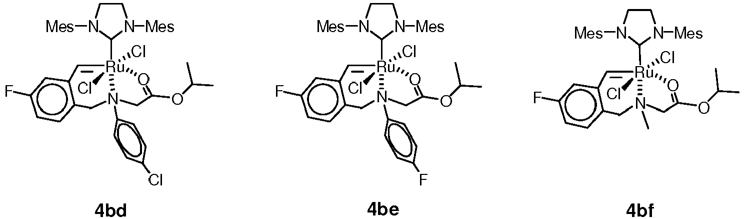

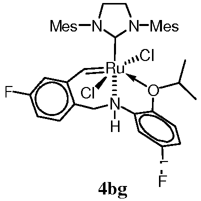

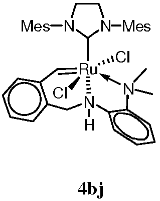

**Two alternative production procedures for preparation of some highly active metathesis catalysts:** In order to prepare different kinds of Ru catalyst at lower cost, based on some references (Zhan et al., US20070043180A1 and WO2007003135A1) and new process development as described in Schemes 9 and 10, there is the following alternative procedure developed in Scheme 9 for scale-up production of different Ru catalysts in the present invention.

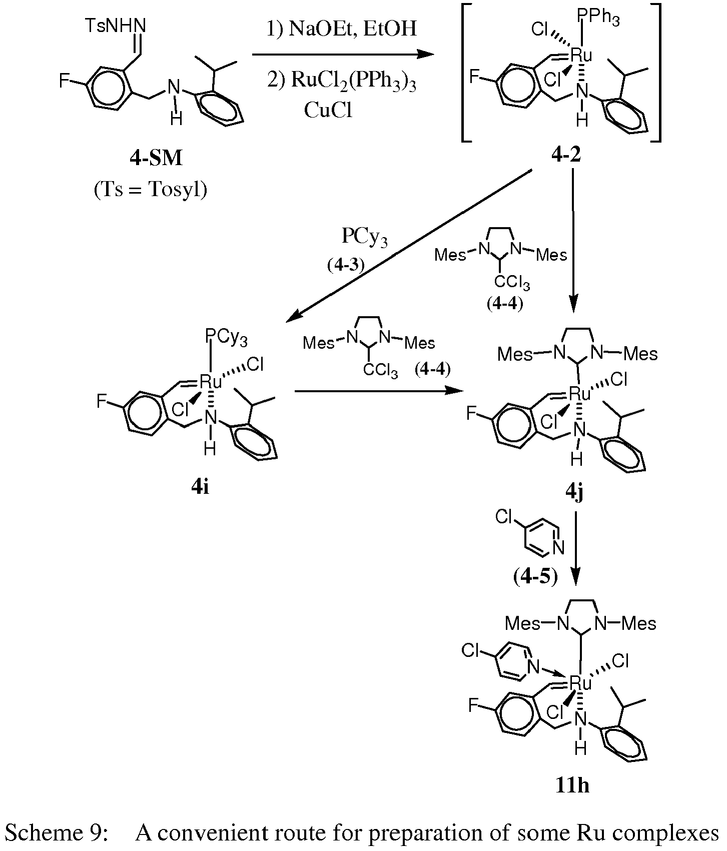

In Scheme 9, the starting material **4-SM** was reacted with sodium ethoxide to produce carbene intermediate **4-1** first, followed by reacting with RuCl₂(PPh₃)₃ directly to form Ru complex intermediate **4-2**. The triphenylphosphine ligand (PPh₃) of Ru intermediate **4-2** was replaced by another ligand PCy₃ (**4-3**, **IIId**) to form a new Ru complex **4i**. The phosphine ligand of Ru intermediate **4-2** or **4i** was further replaced by an NHC ligand (H₂IMes, **4-4**, **IIIa**) to form another Ru complex **4j**. The Ru complex **4j** could directly react with another ligand 4-chloro pyridine (**4-5**) to make the Ru complex **11h.**

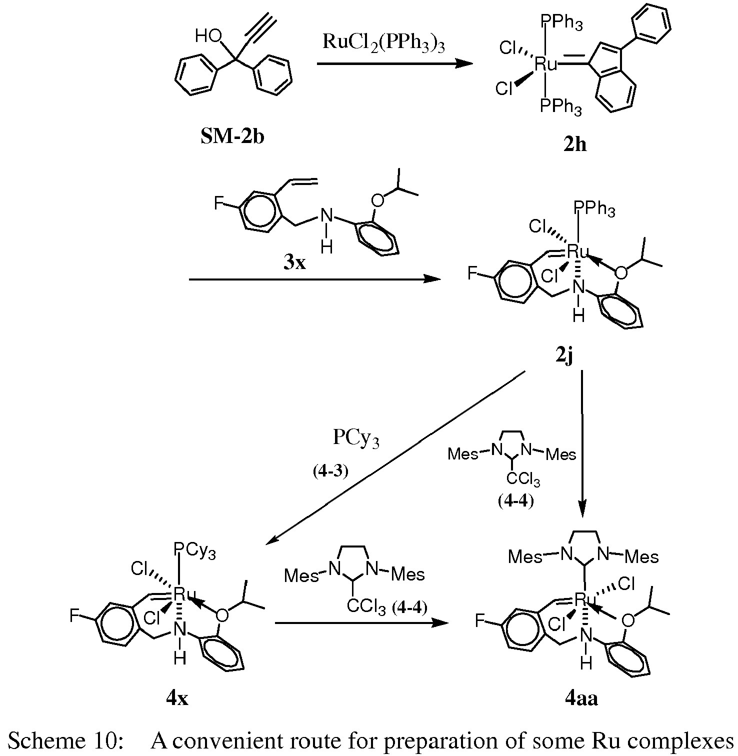

In Scheme 10, The Ru complex **2h** is prepared by the reaction of reagents **SM-2b** and RuCl₂(PPh₃)₃ in anhydrous DCM in a three-neck flask filled with inert gas (Ar), followed by reacting the Ru complex **2h** with complex ligand **3x** (**Ia**) to form another Ru complex **2j** (**Va**) in a flask filled with inert gas (Ar). The triphenylphosphine ligand (PPh₃) of Ru intermediate **2j** was replaced by another phosphine ligand PCy₃ (**4-3, IIId**) to form a new Ru complex **4x**. The phosphine ligand of Ru intermediate **2j** or **4x** was further replaced by another NHC ligand (H₂IMes, **4-4**, **IIIa**) to form another Ru complex **4aa.**

So far, to study the relative activity and catalytic selectivity of above prepared catalysts **4a-4bj**, **6a-6j**, **8a-8u**, **10a-10j**, and **11a-11r**, two olefin substrates **15** and **17** in Equations 1 and 2 were chosen for RCM reactions, and different kinds of cyclic olefin substrates **19**, **21**, **23**, **25**, **27**, **29** and **31** in Equations 3-9 were selected for ROMP reactions, and the kinetic results of different conducted RCM and ROMP reactions for each new catalyst are listed in Tables 1, 2, 3, 4 and 5, respectively. Other eight prior known Ru catalysts **1a-1b and 2a-2f** listed in Scheme 1 are also selected for evaluation of metathesis activity study with various substrates **15**, **17**, **19**, **21**, **23**, **25**, **27**, **29** and **31** in comparison to all new Ru catalysts in the present invention.

The evaluation of catalytic activity for RCM in Equation 1 with different catalysts **4a-4bj**, has been done under the same reaction condition, and the valuable experimental data for different Ru catalysts are selected and listed in Table 1-1.

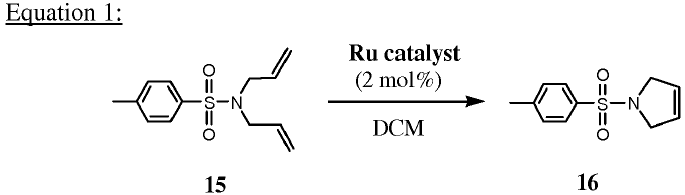

**Table 1-1: Activity Results of Some Selected Complexes 4a-4bj for Substrate 15**

| Entry | Catalyst | Conversion (% by HPLC) | | | |
|---|---|---|---|---|---|
| | | 10 min | 30 min | 1.5 hr | 3.0 hr |
| 1 | **4a** | 40 | 81 | 98 | |
| 2 | **4b** | 52 | 85 | 98 | |
| 3 | **4e** | 0 | 0 | 0 | |
| 4 | **4f** | 84 | 94 | 96 | 100 |
| 5 | **4g** | 94 | 100 | | |
| 6 | **4h** | 3 | 5 | 11 | 16 |
| 7 | **4u** | 96 | 100 | | |
| 8 | **4y** | 43 | 66 | 87 | 94 |
| 9 | **4aa** | 21 | 59 | 82 | 92 |
| 10 | **4ab** | 94 | 100 | | |
| 11 | **4ac** | 70 | 90 | 100 | |
| 12 | **4af** | 93 | 97 | | |
| 13 | **4aj** | 95 | 99 | | |
| 14 | **4ap** | 71 | 82 | 93 | 98 |
| 15 | **4at** | 24 | 48 | 73 | 100 |
| 16 | **4ba** | 62 | 73 | 79 | 85 |
| 17 | **4bb** | 100 | | | |
| 18 | **4bc** | 94 | 100 | | |
| 19 | **4bd** | 68 | 81 | 84 | 89 |
| 20 | **4be** | 100 | | | |

Among Ru complexes **4a-4bj**, only some of new complexes (such as **4f**, **4g**, **4u**, **4ab**, **4aj**, **4bb** and **4be**) show high catalytic activity, the rest of them not listed in Table 1-1 have lower or very poor activities for RCM reaction. Based on the determined results in Table 1-1, the activity of Ru complexes **4a-4bj** for RCM is significantly affected by the electronic and steric effect of different substituents incorporated in various new ligands **3a-3bj.** However, some of complexes **4a-4bj** non-active for RCM can be used effectively in the following ROMP (Equations 3-9) with high activity and selectivity.

In order to find some new catalysts with better activity and selectivity, it is designed to carry out a RCM reaction with a phenyl-substituted diene substrate **17** as shown in Equation 2 instead of unsubstituted diene substrate **15** for further evaluation of some active catalysts selected from the catalysts **4a-4bj** according to activity results in Tables 1-1. The experimental results of RCM activity for substrate **17** are listed in Tables 2.

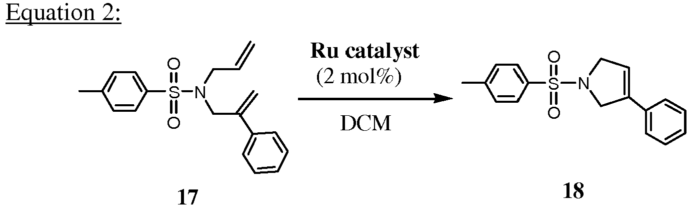

**Table 2: Activity Results of Some Selected Ru Complexes for Substrate 17**

| Entry | Catalyst | Conversion (% by HPLC) | | | |
|---|---|---|---|---|---|
| | | 10 min | 30 min | 1.5 hr | 3.0 hr |
| 1 | **4f** | 67 | 86 | 94 | 99 |
| 2 | **4g** | 61 | 78 | 86 | 98 |
| 5 | **4ab** | 63 | 85 | 96 | 99 |
| 6 | **4ad** | 51 | 68 | 79 | 95 |
| 7 | **4af** | 72 | 86 | 92 | 99 |
| 8 | **4be** | 49 | 73 | 86 | 91 |

To develop more effective ROMP catalysts and prepare better quality of new functional polymers, and also better to measure the difference of various active Ru catalysts, the evaluation of catalytic activity for different ROMP reactions in Equations 3-9 with different catalysts **4a-4bj** has been done under the same reaction condition, and some valuable results for different Ru catalysts are selected or listed in Tables 3 to 6, respectively. Based on the broad test, it is useful to find some active and selective catalysts for ROMP and RCM reactions, respectively.

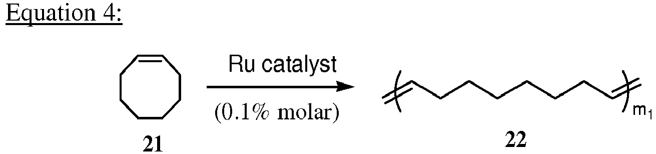

After screening with most of new Ru catalysts, it is found that some catalysts such as **4d** could catalyze the ROMP reaction effectively.

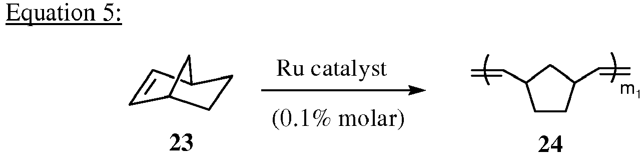

The ROMP results show that the catalysts **4b**, **4f**, **4v**, **4y and 4aa** of the present invention have better activity and selectivity for norbornene (**23**) polymerization. Catalytic polymerization was completed in 10-60 min, and the polymer product (**24**) has better tensile-strength when it is prepared as film.

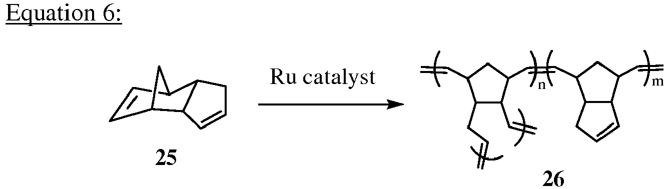

The ROMP results show that the catalysts **4b**, **4f**, **4v**, **4y**, **4aa**, **4ag**, **4ar and 4au** of the present invention have better activity and selectivity for **DCPD** (**25**) polymerization. The ROMP polymerization was completed in 5-60 min for different Ru catalysts. The reaction temperature is preferred to be 40-60°C. By using one or two more mixed catalysts, it is surprised to obtain the high strength and high stiffness polymer PDCPD.

The property tests of various PDCPD (**26**) samples in the present invention show that several PDCPD products have more better tensile strength (55-62Mpa) and flexural strength (78-83Mpa) than those of commercial PDCPD products such as "Pentam, Metton, and Prometa" (tensile strength: 40-50Mpa, and flexural strength: 66-75Mpa) reported by other companies prepared with their own ROMP catalysts in Japan and USA, which advantage in the present invention will provide an alternative method of making high-quality of PDCPD material for broad uses in polymer industry.

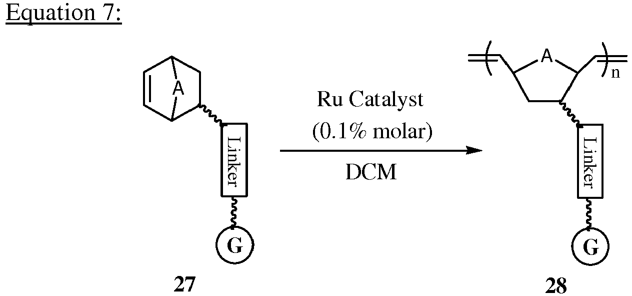

Some selected structure of prepared polymers **28a-28g** is listed as follows, and ROMP results are listed in Table 3:

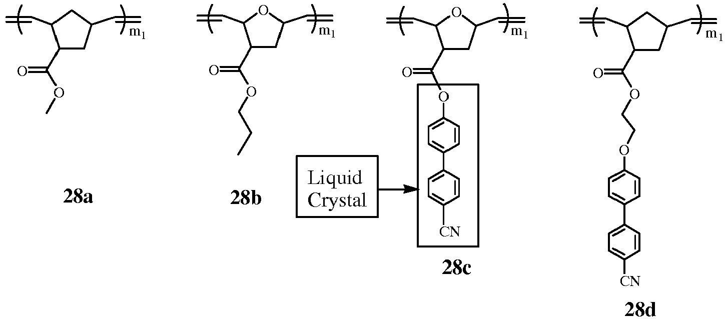

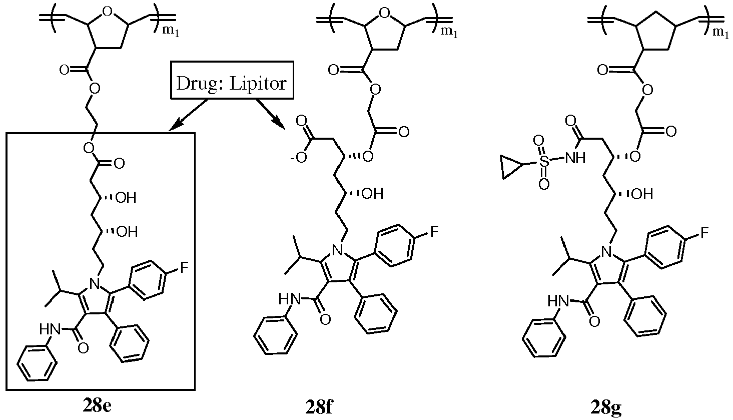

**Table 3: Selected ROMP results**

| **SM** | **Amount** (**g**) | **Solvent** | **Catalyst** (0.1%) | **Yield** (%) | **Polymer** | **Appearance** |
|---|---|---|---|---|---|---|
| **27a** | 0.5 | DCM | **4d** | 90 | **28a** | white solid |
| **27b** | 0.5 | DCM | **4d** | 93 | **28b** | white solid |
| **27c** | 0.5 | DCM | **4d** | 97 | **28c** | white solid |
| **27d** | 0.5 | DCM | **4d** | 95 | **28d** | white solid |
| **27e** | 0.5 | DCM | **4d** | 93 | **28e** | white solid |
| **27f** | 0.5 | DCM | **4d** | 81 | **28f** | white solid |
| **27g** | 0.5 | DCM | **4d** | 87 | **28g** | white solid |

The results of Table 3 show that, small molecule liquid crystal or pro-drug monomer can react with new Ru catalysts selected from the present invention to form polymerized macromolecule liquid crystal (**28c** and **28d**) and polymer-linked prodrugs (**28e**, **28f** and **28g**) with special properties and applications. The results of activity test show that several new catalysts (such as **4d**, **4f**) of the present invention have better catalytic activity for olefin monomers (**27a-27g**), and the ROMP reactions were completed in 5-15 hrs. Yield is better than 80% with optimized polymerization conditions in the presence of new Ru catalyst **4d**.

The results of polymerization test show that different Ru catalysts of the present invention have significantly different activity and selectivity for different cyclo-olefin monomers. In particular, some new Ru catalysts (e.g., **4d**) have lower catalytic activities in RCM reaction, but have very good activity in ROMP reactions, which demonstrates that several new Ru catalysts in the present invention have the high selectivity and catalytic activity for ROMP and RCM, respectively.

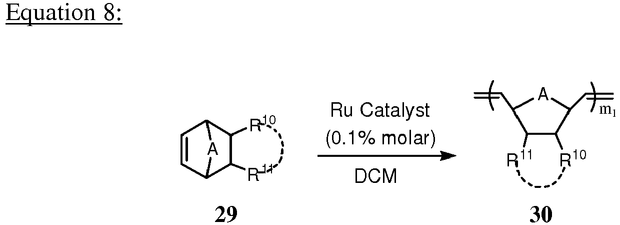

Some selected structure of prepared polymers **30a-30n** is listed as follows, and some selected ROMP results are listed in Table 4:

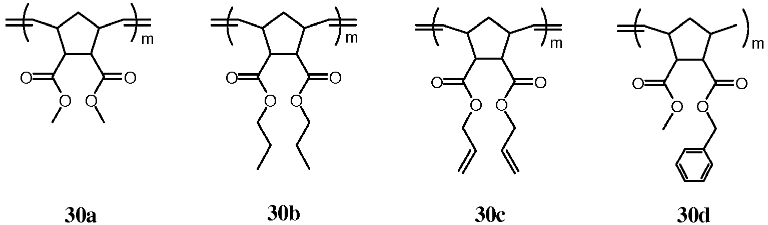

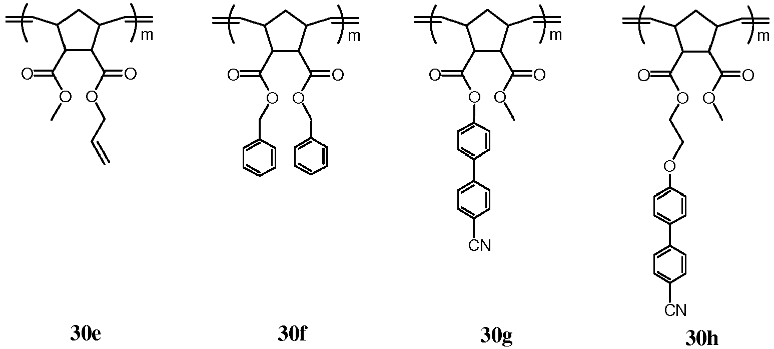

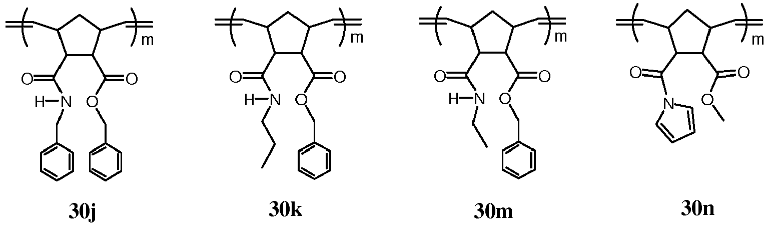

**Table 4: Selected ROMP results**

| **SM** | **Amount** (g) | **Solvent** | **Catalyst** (0.1%) | **Yield** (%) | **Polymer** | **Appearance** |
|---|---|---|---|---|---|---|
| **29a** | 0.5 | DCM | **4d** | 75 | **30a** | white solid |
| **29b** | 0.5 | DCM | **4d** | 90 | **30b** | white solid |
| **29c** | 0.5 | DCM | **4d** | 71 | **30c** | white solid |
| **29d** | 0.5 | DCM | **4d** | 60 | **30d** | white solid |
| | | | | | | |
| **29f** | 0.5 | DCM | **4d** | 85 | **30f** | white solid |
| **29g** | 0.5 | DCM | **4d** | 97 | **30g** | white solid |
| **29h** | 0.5 | DCM | **4d** | 98 | **30h** | white solid |
| **29j** | 0.5 | DCM | **4d** | 97 | **30j** | white solid |
| **92k** | 0.5 | DCM | **4d** | 96 | **30k** | white solid |
| **29m** | 0.5 | DCM | **4d** | 95 | **30m** | white solid |
| **29n** | 0.5 | DCM | **4d** | 93 | **30n** | white solid |

The results in Table 4 show that most of cyclo-olefin monomers with different functional groups (**29a-29n**) were polymerized in the presence of new Ru catalysts such as **4d** selected from the present invention to form functional polymers with different chemical and physical properties.

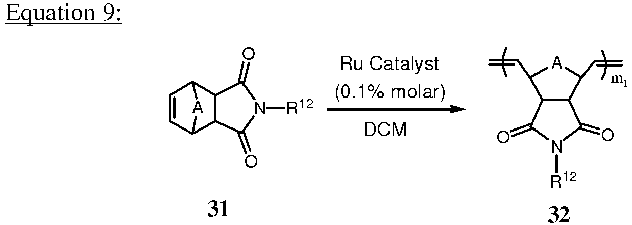

Some selected structure of prepared polymers **32a-32m** is listed as follows, and some selected ROMP results are listed in Table 5:

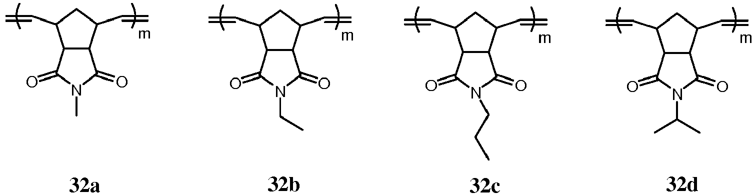

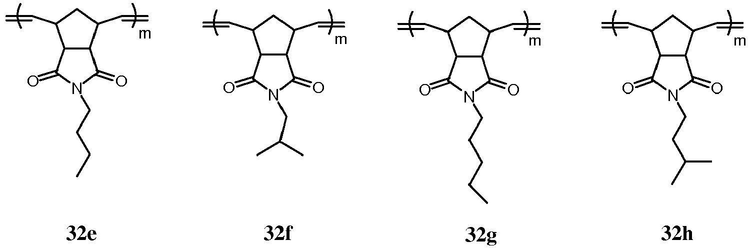

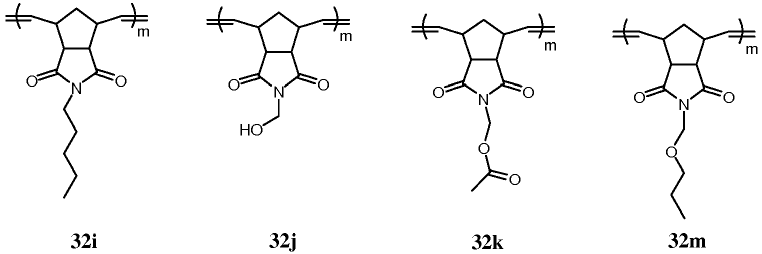

**Table 5: Selected ROMP results**

| **SM** | **Amount (g)** | **Solvent** (mL) | **Catalyst** (0.1%) | **Yield** (%) | **Polymer** | **Appearance** |
|---|---|---|---|---|---|---|
| **31a** | 0.5 | DCM | **4d** | 96 | **32a** | white solid |
| **31b** | 0.5 | DCM | **4d** | 92 | **32b** | white solid |
| **31c** | 0.5 | DCM | **4d** | 92 | **32c** | white solid |
| **31d** | 0.5 | DCM | **4d** | 89 | **32d** | white solid |
| **31e** | 0.5 | DCM | **4d** | 91 | **32e** | white solid |
| **31f** | 0.5 | DCM | **4d** | 87 | **32f** | white solid |
| **31g** | 0.5 | DCM | **4d** | 91 | **32g** | white solid |
| **31h** | 0.5 | DCM | **4d** | 85 | **32h** | white solid |
| **31i** | 0.5 | DCM | **4d** | 87 | **32i** | white solid |
| **31j** | 0.5 | DCM | **4d** | 97 | **32j** | white solid |
| **31k** | 0.5 | DCM | **4d** | 87 | **34k** | white solid |
| **31m** | 0.5 | DCM | **4d** | 98 | **32m** | white solid |

The results in Table 5 show that hat most of cyclo-olefin monomers with different functional groups (**31a-31m**) were polymerized in the presence of new Ru catalyst **4d** selected from the present invention to form functional polymers with different chemical and physical properties. Moreover, several products **32a**, **32b**, **32c**, and **34m** could be used to form film with high strength (over 50 Mpa).

The catalysts of the present invention can be used for depolymerization of a rubber comprising at least one carbon-carbon double bond. The depolymerization is conducted by metathesis reaction of carbon-carbon double bond in the rubber in the presence of one or more of catalysts of the present invention. The depolymerized rubber has lower molecular weight and lower mooney viscosity, which can be better used at lower temperature as lower as -40°C.

The catalysts of the present invention can be used in hydrogenation of a rubber comprising at least one carbon-carbon double bond. The carbon-carbon double bond in the rubber is hydrogenated under high pressure of hydrogen in the presence of one or more of catalysts of the present invention. The hydrogenated rubber is obtained and could be used as more stable and higher strength rubber.

The rubber comprising at least one carbon-carbon double bond can be depolymerized, and followed by hydrogenation under high pressure of hydrogen to produce a lower molecular weight and lower mooney viscosity rubber in the presence of one or more catalysts of the present invention, which can be used at lower temperature as lower as -55°C.

The rubber comprising at least one carbon-carbon double bond can be hydrogenated under high pressure of hydrogen and depolymerized simultaneously in the presence of one or more catalysts of the present invention, which can be used at lower temperature as lower as -55°C.

The representative examples of rubbers include but not limited to nitrile butadiene rubber, polybutadiene rubber, styrene-butadiene rubber (SBR), styrene-butadiene-styrene (SBS) or any rubber containing carbon-carbon double bond.

For example, the NBR is depolymerized by using catalyst **4ab** as shown in Equation 10:, and the physical properties of depolymerized NBR are listed in Table 6. 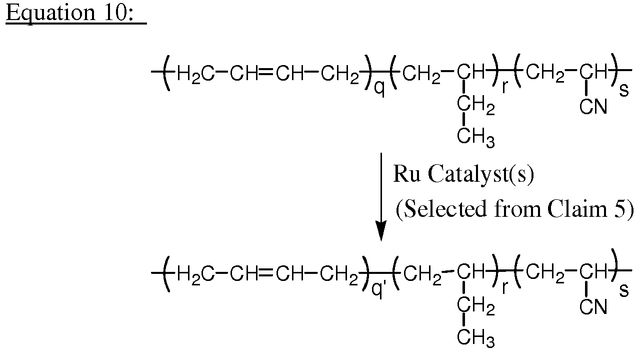 (In Equation, where q > q')

**Table 6: Depolymerization Results by Ru Catalysts**

| **Entry No.** | **Samples** | **Mw** | **Mn** | **Mooney viscosity** |
|---|---|---|---|---|
| 1 | NBR (N41), RM | 4.11E+05 | 1.81E+05 | 77.5 |
| 2 | 0.04 wt% Catalyst (**4ab**) | 2.78E+05 | 1.586E+5 | 60.3 |
| 3 | 0.07 wt% Catalyst (**4ab**) | 2.16E+05 | 1.05E+05 | 54.1 |
| 4 | 0.10 wt% Catalyst (**4ab**) | 1.11E+05 | 7.41E+04 | 37.9 |

| | | | | |
|---|---|---|---|---|
| Notes: Mw and Mn: Molecular weight. RM: Raw Material. | | | | |

So far, it is determined that the molecular weight (Mw) and Mooney viscosity of various nitrile butadiene rubber (e.g., commercially available from Zeon company (Japan) in trade name N41, DN3335, DN3350, and DN2850) are significantly reduced down about 30-70% as needed by metathesis depolymerization in chlorobenzene or chloroform in the presence of catalysts of the present invention (e.g., catalyst **4ab** selected from **4a-4bj**). 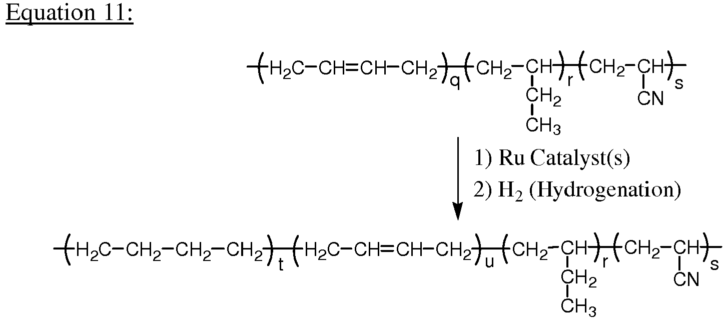 In Equation 11, q > (t + u).

The process for Equation 11 was carried out by adding Ru metathesis catalyst (4aa) for depolymerization first, followed by adding hydrogen for hydrogenation in chlorobenzene. It is determined that the molecular weight (Mw) and Mooney viscosity of various nitrile butadiene rubbers (e.g., commercially available from Zeon company (Japan) in trade name N41, DN3335, DN3350, and DN2850) are significantly reduced down about 30-70% as needed by depolymerization in chlorobenzene or chloroform in the presence of the catalysts of the present invention (e.g., **4a-4bj**), and the hydrogenation degree is determined to be between 90-99.5% as needed. The depolymerization and hydrogenation results are listed in the following Table 7.

**Table 7: Depolymerization and Hydrogenation Results by Ru Catalysts**

| **Entry No.** | **Samples** | **Mw** | **Mn** | **Iodine Value** | **[H] Degree** |
|---|---|---|---|---|---|
| 1 | NBR (N41), RM | 4.11E+05 | 1.81E+05 | 290 | --- |
| 2 | 0.04 wt% Catalyst (**4aa**) | 2.70E+05 | 1.62E+05 | 23.5 | >90% |
| 3 | 0.07 wt% Catalyst (**4aa**) | 1.60E+05 | 1.12E+05 | 12.6 | >95% |
| 4 | 0.10 wt% Catalyst (**4aa**) | 2.10E+04 | 1.32E+04 | 3.5 | >99% |

| | | | | | |
|---|---|---|---|---|---|
| Notes: Mw and Mn: Molecular weight; [H]: Hydrogenation; RM: Raw Material, which is undepolymerized and unhydrogenated. | | | | | |

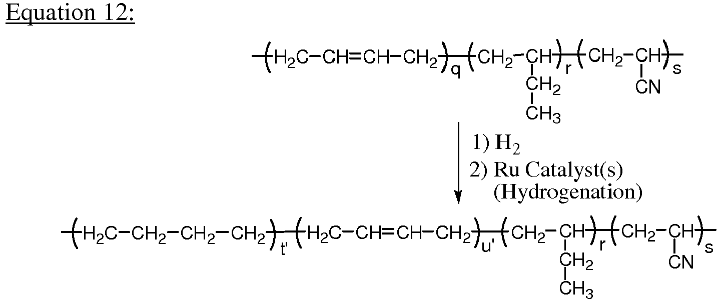

In Equation 12, q > (t'+u').

The process for Equation 12 was carried out by adding hydrogen first, followed by adding Ru metathesis catalyst (**4aa**) to conduct hydrogenation and depolymerization simultaneously in chlorobenzene. It is determined that the hydrogenation degree is determined to be between 90-99.5% as needed (determined by 1HNMR), and the molecular weight (Mw) and Mooney viscosity of various nitrile butadiene rubbers (e.g., commercially available from Zeon company (Japan) in trade name N41, DN3335, DN3350, and DN2850) are reduced down about 10-50% as needed by depolymerization in chlorobenzene or chloroform in the presence of catalysts (e.g., **4a-4bj**). The depolymerization and hydrogenation results are listed in Table 8.

**Table 8: Selected Hydrogenation and Depolymerization Results by Ru Catalysts**

| **Entry No.** | **Samples** | **Mw** | **Mn** | **Iodine Value** | **[H] Degree** |
|---|---|---|---|---|---|
| 1 | NBR (N41), RM | 4.11E+05 | 1.81E+05 | 290 | --- |
| 2 | 0.04 wt% Catalyst (**4aa**) | 3.07E+05 | 1.87E+05 | 15.3 | >95% |
| 3 | 0.07 wt% Catalyst (**4aa**) | 2.10E+05 | 1.18E+05 | 11.8 | >96% |
| 4 | 0.10 wt% Catalyst (**4aa**) | 1.80E+05 | 1.07E+05 | 3.1 | >99% |

| | | | | | |
|---|---|---|---|---|---|
| RM: Raw Material, which is undepolymerized and unhydrogenated. | | | | | |

Based on the results from Equation 10 to Equation 12, it is determined that the molecular weight (Mw) and Mooney viscosity of various NBR brands (e.g., N41, DN3335, DN3350, and DN2850) are obviously reduced about 30-70% as needed by metathesis depolymerization and hydrogenation by adding hydrogen in chlorobenzene or chloroform in the presence of the Ru catalysts (e.g., **4a-4bj**) to obtain different kinds of HNBR products as needed with lower molecular weight (Mooney viscosity range: 20-100MU) and high hydrogenation degree (90-99.5%).

So far, it is found that most of the new developed Ru catalysts (**4a-4bj**) can be used to reduce molecular weight of the nitrile butadiene rubber (NBR) and butyl rubber by catalytical depolymerization. Furthermore, the quality-modified hydrogenated nitrile butadiene rubber (HNBR) with different molecular weight has been prepared by adding different new Ru catalyst and hydrogen (H₂) under high pressure (2.0-15Mpa) in some organic solvents such as chlorobenzene or chloroform solution. Just as mentioned above, the depolymerized NBR can be used in lower temperature as lower as -40°C, and the depolymerized and hydrogenated NBR (HNBR) can be used in a temperature as lower as -55°C with an improved strength and a better UV-resistance.

Based on our broad study, it is found that some of novel Ru catalysts (such as **4a-4bj**) have good activity for metathesis depolymerization to prepare different kinds of lower molecular NBR, followed by hydrogenation under high pressure of hydrogen (preferred between 4-9Mpa) to prepare high hydrogenation degree and various molecular weight of HNBR products.

Overall, based on the activity and selectivity studies in equations 1-10, it is found that some of novel Ru catalysts such as **4d**, **4f**, **4g and 4ab**, have much better activity and selectivity than other tested and reported metathesis catalysts for the ROMP and RCM reactions, respectively. Moreover, it is found that the electronic effect of multi-substituted benzylidene ligands on the activity and selectivity of Ru complexes is one of the most important factors for the development of new active and selective metathesis catalysts for ROMP and RCM reactions. Based on the intensive study, the present invention provides some useful methods of carrying out ROMP, RCM, CM, and ADMET reactions with one or two more mixed of novel active Ru catalysts for preparation of some functional polymers, lower molecular weight rubbers and/or pharmaceutical intermediates, respectively.

### EXAMPLES

**General:** Infrared (IR) spectra were recorded on a Fourier Transform AVATAR™ 360 E.S.P™ spectrophotometer (Unit: cm⁻¹). Bands are characterized as broad (br), strong (s), medium (m), and weak (w). ¹H NMR spectra were recorded on a Varian-400 (400 MHz) spectrometer. Chemical shifts are reported in ppm from tetramethylsilane with the solvent resonance as the internal standard (CDCl₃: 7.26 ppm). Data are reported as follows: chemical shift, multiplicity (s = singlet, d = doublet, t = triplet, q = quartet, br = broad, m = multiplet), coupling constants (Hz), integration, and assignment. ¹⁹F and ³¹P NMR spectra were recorded on a Varian-400 (400 MHz) and Gemini-2000 (300MHz) spectrometers. The chemical shifts of the fluoro resonances were determined relative to trifluoroacetic acid as the external standard (CF₃CO₂H: 0.00 ppm), and the chemical shifts of the phosphorus resonances were determined relative to phosphoric acid as the external standard (H₃PO₄: 0.00 ppm). Mass spectra were obtained at Thermo Finnigan LCQ Advantage. Unless otherwise noted, all reactions were conducted in oven- (135°C) and flame-dried glassware with vacuum-line techniques under an inert atmosphere of dry Ar. THF and Et₂O were distilled from sodium metal dried flask, DCM, pentane, and hexanes were distilled from calcium hydride. Different substituted 2-alkoxystyrene ligands were prepared according to literature procedures as shown in Schemes 1-3. SM-Ia and SM-Ib chemicals were obtained from commercial sources or ordered by custom synthesis from Zannan Pharma Ltd., China. General procedures for preparation of different Ru complexes are described in examples 1 and 2, respectively. General procedures for evaluation of the RCM and ROMP reactions are described in examples 104-107, respectively.

### Example 1

### Synthesis of Ru complex 4a

SM-**3a** (5.0mmol) was added into a 50 mL of three-neck round-bottom flask filled with inert gas (Ar), and followed by adding DME (10 mL) and deionized water (3 mL). K₂CO₃ (1.5 eq) was added and the solution was N₂ protected. The reaction was heated to 85°C, 2,4,6-Trivinyl-cyclotriboroxane pyridine complex (0.5 eq) and Pd(PPh₃)₄ (2%) were added until completed overnight. (monitored by TLC). The reaction mixture was filtered and extracted by DCM twice, then purified by flash column eluting with a gradient solvent (PE/EA 400/1 to 100/1) and dried under vacuum to obtain 0.9g of yellow oil products **3a** (yield: 86%). The product was confirmed by ¹HNMR.

Ligand **3a** ¹H-NMR (400 MHz, CDCl₃): δ 7.56 (d, *J* = 7.5 Hz, 1H, aromatic H), 7.37-7.18 (m, 5H, aromatic H, CH=CH₂), 7.02 (dd, *J* = 17.4 Hz, 10.8 Hz, 1H, CH=CH₂), 6.76-6.64 (m, 3H, aromatic H), 5.72 (d, *J* = 17.4 Hz, 1H, CH=CH₂), 5.34 (d, *J=* 10.8 Hz, 1H, CH=CH₂), 4.33 (s, 2H, NCH₂), 3.83 (s, 1H, NH).

(H₂IMes)(PCy₃)Cl₂Ru=CHPh (formula **1b**, 860mg, 1.0mmol) and CuCl (270 mg, 2.5mmol, 2.5 eq) were added into a 100 mL of two-neck round-bottom flask filled with inert gas (Ar), and followed by adding DCM (15 mL) and ligand **3a** (250 mg, 1.2mmol, 1.2 eq) into the DCM solution at 20-25°C. The reaction was stirred until completed in 30-60 min. (monitored by TLC). The reaction mixture was filtered and concentrated, then purified by flash column eluting with a gradient solvent (Pentane/DCM 2/1 to DCM). The purified solid product was washed with methanol, and dried under vacuum to obtain 27mg of green solid product **4a**, yield: 4%. The green product was confirmed by ¹HNMR.

Ru complex **(4a)** ¹HNMR (400 MHz, CDCl₃): δ 19.09 (s,1H, Ru=CH), 7.51-6.70 (m, 13H), 5.31 (m, 1H), 4.30 (d, *J* = 12.9 Hz, 1H), 4.04 (s, 4H, NCH₂CH₂N), 3.61 (d, *J* = 12.9 Hz, 1H), 2.45 (s, 12H), 2.33 (s, 6H).

### Example 2

### Synthesis of Ru complex 4b

The synthetic procedure for preparation of ligand **3b** is the same as in Example 1 in 5.0 mmol scale. 1.15g of yellow oil product **3b** was obtained (yield: 91%).

Ligand **3b** ¹H-NMR (400 MHz, CDCl₃): δ 7.54 (d, *J* = 6.6 Hz, 1H), 7.27-7.23 (m, 3H), 7.02 (dd, *J* = 11.1 Hz, 17.0 Hz, 1H), 6.87-6.84 (m, 2H), 6.75-6.72 (m, 2H), 5.69 (dd, *J* = 1.5 Hz, 17.0 Hz, 1H), 5.35 (dd, *J* = 1.5 Hz, 11.1 Hz, 1H), 4.47 (s, 2H), 3.78 (s, 3H), 2.93 (s, 3H).

(PCy₃)₂Cl₂Ru=CHPh (formula **1a**, 830mg, 1.0mmol) and CuCl (270 mg, 2.5mmol, 2.5 eq) were added into a 100 mL of two-neck round-bottom flask filled with inert gas (Ar), and followed by adding DCM (15 mL) and ligand **3b** (250 mg, 1.2mmol, 1.2 eq) into the DCM solution at 20-25°C. The reaction was stirred until completed in 30-60 min. (monitored by TLC). The reaction mixture was filtered and concentrated, then purified by flash column eluting with a gradient solvent (Pentane/DCM 2/1 to DCM). The purified solid product was washed with methanol, and dried under vacuum to obtain 195mg of green solid product **4b**, yield: 29%. The green product was confirmed by ¹HNMR.

Ru complex **(4b)** ¹HNMR (400 MHz, CDCl₃): δ 19.31 (d, *J* = 8.4 Hz, Ru=CH), 7.57-7.50 (m, 4H), 7.31-7.29 (m, 1H), 7.15 (d, *J* = 5.6 Hz, 1H), 6.84-6.81 (m, 2H), 5.78 (d, *J* = 12.0 Hz, 1H), 3.71 (s, 3H), 3.62 (d, *J* = 12.0 Hz, 1H), 2.51 (s, 3H), 2.22-1.13 (m, 33H, PCy₃).

### Example 3

### Synthesis of Ru complex 4c

The synthetic procedure for preparation of ligand **3c** is the same as in Example 1 in 5.0 mmol scale. 0.66g of yellow oil product **3c** was obtained (yield: 54%).

Ligand **3c** ¹H-NMR (400 MHz, CDCl₃): δ 7.56 (d, *J* = 7.5 Hz, 1H, aromatic H), 7.34-7.26 (m, 3H, aromatic H, CH=CH₂), 7.13 (d, *J =* 9 Hz, 1H, CH=CH₂), 6.98 (dd, *J* = 17.4 Hz, 10.8 Hz, 1H, CH=CH₂), 6.56 (d, *J* = 9 Hz, 1H, CH=CH₂), 5.71 (dd, *J* = 17.4 Hz, 1.2 Hz, 1H, CH=CH₂), 5.35 (dd, *J* = 10.8 Hz, 1.2 Hz, 1H, CH=CH₂), 4.30 (s, 2H, NCH₂), 3.86 (s, 1H, NH).

The procedure for preparation of Ru complex **4c** is the same as in Example 1 in 1.0 mmol scale. 35 mg of green solid product **4c** was obtained (yield: 5%).

Ru complex **(4c)** ¹HNMR (400 MHz): δ 19.09 (s,1H, Ru=CH), 7.50-6.69 (m, 12H), 5.27 (m, 1H), 4.33 (d, *J* = 12.9 Hz, 1H), 4.04 (s, 4H, NCH₂CH₂N), 3.59 (d, *J* = 12.9 Hz, 1H), 2.45 (s, 12H), 2.37 (s, 6H).

### Example 4

### Synthesis of Ru complex 4d

The synthetic procedure for preparation of ligand **3d** is the same as in Example 1 in 5.0 mmol scale. 0.74g of yellow oil product **3d** was obtained (yield: 62%).

Ligand **3d** ¹H-NMR (400 MHz, CDCl₃): δ 7.32-7.23 (m, 2H), 7.04-6.91 (m, 2H), 6.82 (dd, *J* = 2.0 Hz, 6.6 Hz, 2H), 6.62 (dd, *J* = 2.4 Hz, 6.6 Hz, 2H), 5.73 (d, *J* = 17.1 Hz, 1H), 5.39 (d, *J=* 11.1 Hz, 1H), 4.25 (s, 2H), 3.77 (s, 3H).

The procedure for preparation of Ru complex **4d** is the same as in Example 1 in 1.0 mmol scale. 231 mg of green solid product **4d** was obtained (yield: 32%).

Ru complex **(4d)** ¹HNMR (400 MHz, CDCl₃): δ 18.68 (s, Ru=CH), 7.23-6.65 (m, 10H), 6.36 (dd, *J* = 2.8, 9.6 Hz, 1H), 6.03 (d, *J* = 12.8 Hz, 1H), 4.14-3.90 (m, 4H, NCH₂CH₂N), 3.85 (s, 3H), 3.47 (d, *J* = 12.8 Hz, 1H), 2.89-1.62 (m, 18H).

### Example 5

### Synthesis of Ru complex 4e

The structure of ligand **3e** is the same as **3d** for preparation of Ru complex **4e,** just another Ru complex reagent **1a** was used instead of Ru reagent **1b.**

The procedure for preparation of Ru complex **4e** is the same as in Example 2 in 1.0 mmol scale. 243 mg of green solid product **4e** was obtained (35% yield).

Ru complex **(4e)** ¹HNMR (400 MHz, CDCl₃): δ 19.28 (d, *J* = 8.4 Hz, Ru=CH), 7.45 (d, *J* = 8.8 Hz, 2H), 7.31-7.16 (m, 3H), 6.83 (d, *J* = 8.8 Hz, 2H), 5.13 (t, *J* = 12.4 Hz, 1H), 7.96 (d, *J* = 12.4 Hz, 1H), 3.85 (d, *J* = 12.4 Hz, 1H), 3.80 (s, 3H), 2.28-1.24 (m, 33H, PCy₃).

### Example 6

### Synthesis of Ru complex 4f

The synthetic procedure for preparation of ligand **3f** is the same as in Example 1 in 5.0 mmol scale. 0.79g of yellow oil product **3f** was obtained (yield: 63%).

Ligand **3f** ¹H-NMR (400 MHz, CDCl₃): δ 7.21 (m, 2H), 6.94 (m, 2H), 6.85 (m, 2H), 6.73 (m, 2H), 5.68 (dd, *J* = 1.2 Hz, 16.8 Hz, 1H), 5.38 (dd, *J* = 1.5 Hz, 11.4 Hz, 1H), 4.40 (s, 2H), 3.77 (s, 3H), 2.89 (s, 3H).

The procedure for preparation of Ru complex **4f** is the same as in Example 1 in 1.0 mmol scale. 103 mg of green solid product **4f** was obtained (yield: 14%).

Ru complex **(4f)** ¹HNMR (400 MHz, CDCl₃): δ 18.99 (s, Ru=CH), 7.48-7.44 (m, 1H), 7.19-6.86 (m, 7H), 6.72-6.66 (m, 1H), 5.29 (t, *J* = 13.2 Hz, 1H), 4.19-3.58 (m, 8H), 2.52-2.37 (m, 18H).

### Example 7

### Synthesis of Ru complex 4g

The synthetic procedure for preparation of ligand **3g** is the same as in Example 1 in 5.0 mmol scale. 0.70g of yellow oil product **3g** was obtained (yield: 56%). The product **3g** is confirmed by LC-MS (M+H⁺): m/z calculated: 285.1, found: 285.1, and directly used for preparation of Ru complex **4g.**

The procedure for preparation of Ru complex **4g** is the same as in Example 1 in 1.0 mmol scale. 61 mg of green solid product **4g** was obtained (yield: 8%).

Ru complex **(4g)** ¹HNMR (400 MHz, CDCl₃): δ 19.11 (s,1H, Ru=CH), 8.36 (dd, *J* = 2.0, 8.0 Hz, 1H), 7.29-6.65 (m, 10H ), 5.30 (t, *J* = 13.6 Hz, 1H), 4.23 (d, *J* = 13.2 Hz, 1H), 4.10 (s, 3H), 3.80 (s, 4H, NCH₂CH₂N), 3.69 (d, *J* = 13.2 Hz, 1H), 2.65-2.08 (m, 18H).

### Example 8

### Synthesis of Ru complex 4h

The synthetic procedure for preparation of ligand **3h** is the same as in Example 1 in 5.0 mmol scale. 0.47g of yellow solid product **3h** was obtained (yield: 32%).

Ligand **3h** ¹H-NMR (400 MHz, CDCl₃): δ 7.32 (dd, *J* = 5.6 Hz, 8.0 Hz, 1H), 7.25 (dd, *J* = 2.8 Hz, 10.4 Hz, 1H), 7.00-6.92 (m, 2H), 6.34 (s, 2H), 5.72 (d, *J* = 17.2 Hz, 1H), 5.38 (d, *J* = 11.2 Hz, 1H), 4.23 (s, 2H), 3.68 (s, 3H), 2.24 (s, 6H).

The procedure for preparation of Ru complex **4h** is the same as in Example 1 in 1.0 mmol scale. 315 mg of green solid product **4h** was obtained (yield: 42%).

Ru complex **(4h)** ¹HNMR (400 MHz, CDCl₃): 19.02 (s, 1H, Ru=CH), 7.21-6.82 (m, 8H), 6.40 (dd, *J* = 9.6 Hz, 1.6 Hz), 5.21 (m, 1H), 4.06-4.00 (m, 5H), 3.70 (s, 3H), 3.54 (d, *J* = 13.2 Hz, 1H), 2.48-2.18 (m, 24H).

### Example 9

### Synthesis of Ru complex 4j

The synthetic procedure for preparation of ligand **3j** is the same as in Example 1 in 5.0 mmol scale. 0.91g of yellow liquid product **3j** was obtained (yield: 93%).

Ligand **3j** ¹H-NMR (400 MHz, CDCl₃): δ 7.34-7.26 (m, 2H), 7.22-7.13 (m 2H), 6.98-6.95 (m, 2H), 6.81 (m, 1H), 6.70-6.68 (m, 1H), 5.73 (d, *J* = 17.2 Hz, 1H), 5.36 (d, *J* = 11.2 Hz, 1H), 4.32 (s, 2H), 2.81 (m, 1H), 1.24 (d, *J* = 6.8 Hz, 6H).

The procedure for preparation of Ru complex **4j** is the same as in Example 1 in 1.0 mmol scale. 353 mg of green solid product **4j** was obtained (yield: 48%).

Ru complex (**4j**) ¹H-NMR (400 MHz, CDCl₃): δ 18.88 (s,1H, Ru=CH), 7.57-6.44 (m, 11H), 5.36 (t, *J* = 13.2 Hz, 1H), 4.16-4.02 (m, 5H), 4.01 (d, *J* = 13.2 Hz, 1H), 2.75-2.00 (m, 19H), 1.01-0.90 (m, 6H).

### Example 10

### Synthesis of Ru complex 4k

The synthetic procedure for preparation of ligand **3k** is the same as in Example 1 in 5.0 mmol scale. 0.57g of yellow oil product **3k** was obtained (yield: 83%).

Ligand **3k** ¹H-NMR (400 MHz, CDCl₃): δ = 7.26 Hz) : 7.317 (dd, 1H, *J* = 6 Hz,8.4 Hz), 7.256 (dd, *J* = 2.8 Hz, 10.4 Hz, 1H), 7.094-7.017 (m, 3H), 6.961 (td, *J* = 2.8 Hz, 8.8 Hz, 1H), 6.873 (t, 1H, *J* = 6.8 Hz), 5.735 (d, *J* = 17.2 Hz 1H,), 5.412 (d, *J* = 10.8 Hz, 1H), 4.133(s, 2H), 2.276(s, 6H).

The procedure for preparation of Ru complex **4k** is the same as in Example 1 in 1.0 mmol scale. 490 mg of green solid product **4k** was obtained (yield: 68%).

Ru complex **(4k)** ¹HNMR (400 MHz, CDCl₃): δ 18.90 (s, 1H, Ru=CH), 7.27-6.77 (m, 9H), 6.41 (d, *J* = 8.0 Hz, 1H), 5.43 (t, *J* = 13.2 Hz, 1H), 4.18-4.00 (m, 5H), 3.25 (d, J = 13.6 Hz, 1H), 2.76-1.27 (m, 24H).

### Example 11

### Synthesis of Ru complex 4m

The synthetic procedure for preparation of ligand **3m** is the same as in Example 1 in 5.0 mmol scale. 0.76g of yellow oil product **3m** was obtained (yield: 49%). The product **3m** is confirmed by LC-MS (M+H⁺): m/z calculated: 311.2, found: 311.2, and directly used for preparation of the Ru complex **4m.**

The procedure for preparation of Ru complex **4m** is the same as in Example 1 in 1.0 mmol scale. 404 mg of green solid product **4m** was obtained (yield: 52%).

Ru complex **(4m)** ¹HNMR (400 MHz, CDCl₃): δ 18.95 (s,1H, Ru=CH), 7.43-6.36 (m, 10H), 4.00 (m, 6H), 2.67-2.06 (m, 20H), 0.90-0.83 (m, 12H).

### Example 12

### Synthesis of Ru complex 4n

The synthetic procedure for preparation of ligand **3n** is the same as in Example 1 in 5.0 mmol scale. 0.63g of yellow oil product **3n** was obtained (yield: 45%).

Ligand **3n** ¹H-NMR (400 MHz, CDCl₃): δ 7.33 (dd, *J* = 6.8 Hz, 6.8 Hz, 1H), 7.26 (d, *J* = 11.6 Hz, 1H), 7.08 (dd, *J* = 10.8 Hz, 17.6 Hz, 1H), 6.69 (t, *J* = 8.4 Hz, 1H), 6.86 (s, 2H), 5.74 (d, *J* = 17.6 Hz, 1H), 5.42 (d, *J* = 10.8 Hz, 1H), 4.08 (s, 2H), 2.25 (s, 9H).

The procedure for preparation of Ru complex **4n** is the same as in Example 1 in 1.0 mmol scale. 470 mg of green solid product **4n** was obtained (yield: 64%).

Ru complex **(4n):** ¹H-NMR (400 MHz, CDCl₃): δ 18.88 (s,1H, Ru=CH), 7.25-6.36 (m, 9H), 5.40 (t, *J* = 13.2 Hz, 1H), 4.14-4.00 (m, 6H), 2.77-1.90 (m, 27H).

### Example 13

Synthesis of Ru complex **4p**

The synthetic procedure for preparation of ligand **3p** is the same as in Example 1 in 5.0 mmol scale. 0.85g of yellow oil product **3p** was obtained (yield: 67%).

Ligand **3p** ¹H-NMR (400 MHz, CDCl₃): δ=7.26Hz):7.368 (dd, 1H, *J*=6.00 Hz, 8.40 Hz), 7.258-7.126 (m, 4H), 7.019-6.922 (m, 3H), 5.632 (dd, 1H, *J =* 1.20 Hz, 17.60 Hz), 5.287 (dd, 1H, *J* = 1.20 Hz, 11.20 Hz), 4.072 (s, 2H), 2. 537 (s, 3H), 2.290 (s, 3H)

The procedure for preparation of Ru complex **4p** is the same as in Example 1 in 1.0 mmol scale. 184 mg of green solid product **4p** was obtained (yield: 26%).

Ru complex **(4p)** ¹HNMR (400 MHz, CDCl₃): δ 18.91 (s,1H, Ru=CH), 7.63-6.42 (m, 10H), 5.27 (t, *J* = 13.2 Hz, 1H), 4.13-4.01 (m, 5H), 3.44 (d, *J* = 13.2 Hz, 1H), 2.46-2.00 (m, 21H).

### Example 14

### Synthesis of Ru complex 4q

The synthetic procedure for preparation of ligand **3q** is the same as in Example 1 in 5.0 mmol scale. 0.69g of yellow oil product **3q** was obtained (yield: 46%).

Ligand **3q** ¹H-NMR (400 MHz, CDCl₃): δ 7.21 (dd, *J* = 2.8 Hz, 10.0 Hz, 1H), 7.15 (dd, *J* = 5.6 Hz, 7.6 Hz, 1H), 6.97-6.88 (m, 2H), 6.39 (s, 2H), 5.68 (d, *J* = 17.2 Hz, 1H), 5.36 (dd, *J* = 0.8 Hz, 11.2 Hz, 1H), 4.40 (s, 2H), 3.67 (s, 3H), 2.87 (s, 3H), 2.24 (s, 6H).

The procedure for preparation of Ru complex **4q** is the same as in Example 1 in 1.0 mmol scale. 291 mg of green solid product **4q** was obtained (yield: 38%).

Ru complex **(4q)** ¹HNMR (400 MHz, CDCl₃): δ 18.75 (s,1H, Ru=CH), 7.26-6.21 (m, 9H), 4.05-3.85 (m, 5H), 3.72 (s, 3H), 3.34 (d, *J* = 13.2 Hz, 1H), 2.82-0.95 (m, 30H).

### Example 15

### Synthesis of Ru complex 4r

The synthetic procedure for preparation of ligand **3r** is the same as in Example 1 in 5.0 mmol scale. 0.55g of yellow oil product **3r** was obtained (yield: 44%).

Ligand **3r** ¹H-NMR (400 MHz, CDCl₃): δ 7.33-7.25 (m, 2H), 7.00-6.93 (m, 2H), 6.84 (bd, *J* = 8.4 Hz, 2H), 6.55 (dd, *J* = 4.4 Hz, 9.6 Hz, 1H), 5.74 (d, *J* = 17.2 Hz, 1H), 5.40 (d, *J* = 11.2 Hz, 1H), 4.29 (s,2H), 3.46 (bs, 1H), 2.12 (s, 3H).

The procedure for preparation of Ru complex **4r** is the same as in Example 1 in 1.0 mmol scale. 101 mg of green solid product **4r** was obtained (yield: 14%).

Ru complex **(4r)** ¹HNMR (400 MHz, CDCl₃): δ 18.89 (s,1H, Ru=CH), 7.69-6.43 (m, 10H), 5.23 (dd, *J* = 13.2, 11.3 Hz, 1H), 4.16-3.94 (m, 5H), 3.46 (d, *J* = 11.3 Hz, 1H), 2.62-1.00 (m, 21H).

### Example 16

### Synthesis of Ru complex 4s

The synthetic procedure for preparation of ligand **3s** is the same as in Example 1 in 5.0 mmol scale. 0.83g of yellow oil product **3s** was obtained (yield: 51%).

Ligand **3s** ¹H-NMR (400 MHz, CDCl₃): δ 7.30 (dd, *J* = 6.0 Hz, 8.5 Hz, 1H), 7.23 (dd, *J* = 3.0 Hz, 10.0 Hz, 1H), 6.70-6.90 (m, 2H), 6.79 (d, *J* = 8.5 Hz, 2H), 6.58 (d, *J* = 8.5 Hz, 2H), 5.70 (d, *J* = 18.0 Hz, 1H), 5.37 (d, *J* = 11.0 Hz, 1H), 4.23 (s, 2H), 3.88 (t, *J* = 6.5 Hz, 2H) , 1.73 (m, 2H), 1.44 (m, 2H), 1.35-1.31 (m, 4H), 0.90 (t, *J* = 6.0 Hz, 3H).

The procedure for preparation of Ru complex **4s** is the same as in Example 1 in 1.0 mmol scale. 679 mg of green solid product **4s** was obtained (yield: 85%).

Ru complex **(4s)** ¹HNMR (400 MHz, CDCl₃): δ 18.68 (s,1H, Ru=CH), 7.28-6.42 (m, 10H), 6.37 (d, *J* = 8.5 Hz, 1H), 5.05 (m, 1H), 4.06-3.93 (m, 7H,), 3.57 (d, *J* = 12.8 Hz, 1H), 2.89-1.29 (m, 29H).

### Example 17

### Synthesis of Ru complex 4t

The synthetic procedure for preparation of ligand **3t** is the same as in Example 1 in 5.0 mmol scale. 0.67g of yellow product **3t** was obtained (yield: 38%). The product **3t** is confirmed by LC-MS (M+H⁺): m/z calculated: 339.2, found: 339.2, and directly used for preparation of the Ru complex **4t.**

The procedure for preparation of Ru complex **4t** is the same as in Example 1 in 1.0 mmol scale. 185 mg of green solid product **4t** was obtained (yield: 23%).

Ru complex **(4t)** ¹HNMR (300 MHz, CDCl3): δ 18.97 (s,1H, Ru=CH), 8.54-8.45 (m, 2H), 6.66-6.96 (m, 8H), 4.16-4.10 (m, 1H), 4.03 (s, 4H, NCH₂CH₂N), 2.63-1.75 (m, 22H), 0.92 (d, *J* = 7.6Hz), 0.83 (d, *J* = 7.6Hz).

### Example 18

### Synthesis of Ru complex 4u

The synthetic procedure for preparation of ligand **3u** is the same as in Example 1 in 5.0 mmol scale. 0.39g of yellow oil product **3u** was obtained (yield: 28%).

Ligand **3u** ¹H-NMR (400 MHz, CDCl₃): δ 7.53 (d, *J* = 2.0 Hz, 1H), 7.39 (d, *J* = 8.4 Hz, 1H), 7.28-7.26 (m, 1H), 7.21-7.12 (m, 3H), 7.03 (dd, *J* = 10.8 Hz, 17.6Hz, 1H), 5.73 (d, *J* = 17.6 Hz, 1H), 5.43 (d, *J* = 10.8 Hz, 1H), 4.07 (s, 2H), 3.26 (m, 2H), 1.25 (d, *J* = 6.4 Hz, 12 H).

The procedure for preparation of Ru complex **4u** is the same as in Example 1 in 1.0 mmol scale. 254 mg of green solid product **4u** was obtained (yield: 32%).

Ru complex **(4u)** ¹HNMR (300 MHz, CDCl₃): δ 19.03 (s,1H, Ru=CH), 7.48-6.63 (m, 10H), 5.53 (m, 1H), 4.81-4.78 (m, 1H), 4.00 (s, 4H, NCH₂CH₂N), 2.51-2.49 (m, 1H), 2.51-2.32 (m, 18H), 1.12 (d, *J* = 7.6Hz), 1.04 (d, *J* = 7.6Hz).

### Example 19

### Synthesis of Ru complex 4v

The synthetic procedure for preparation of ligand **3v** is the same as in Example 1 in 5.0 mmol scale. 1.08g of yellow oil product **3v** was obtained (yield: 81%).

Ligand **3v** ¹H-NMR (400 MHz, CDCl₃): δ 7.56 (d, *J* = 7.2 Hz, 1H), 7.34 (dd, *J* = 1.6 Hz, 7.6 Hz, 1H), 7.30-7.26 (m, 2H), 7.03 (dd, *J* = 11.2 Hz, 17.2 Hz, 1H), 6.86-6.80 (m, 2H), 6.68-6.62 (m, 2H), 5.72 (dd, *J* = 1.2 Hz, 17.2 Hz, 1H), 5.33 (dd, *J* = 1.2 Hz, 11.2 Hz, 1H), 4.56 (m, 1H), 4.36 (s, 2H), 1.33 (d, *J* = 6 Hz, 6H).

The procedure for preparation of Ru complex **4v** is the same as in Example 1 in 1.0 mmol scale. 73 mg of green solid product **4v** was obtained (yield: 10%).

Ru complex **(4v)** ¹HNMR (400 MHz, CDCl₃): δ 18.97 (s, Ru=CH), 7.50-6.58 (m, 11H), 5.26-3.52 (m, 8H), 3.48-2.07 (m, 18H), 1.23 (d, *J* = 6.4 Hz, 6H).

### Example 20

### Synthesis of Ru complex 4w

The structure of ligand **3w** is the same as **3v** for preparation of Ru complex **4w,** just another Ru complex reagent **1a** was used instead of Ru reagent **1b.**

The procedure for preparation of Ru complex **4w** is the same as in Example 2 in 1.0 mmol scale. 219 mg of green solid product **4w** was obtained (yield: 31%).

Ru complex **(4w)** ¹HNMR (400 MHz, CDCl₃): δ 19.56 (d, *J* = 9.9 Hz, Ru=CH), 8.20 (d, *J* = 8.1 Hz, 1H), 7.66-6.84 (m, 6H), 5.46 (d, *J* = 12 Hz, 1H), 5.22 (t, *J* = 6 Hz, 1H), 4.56 (m, 1H), 3.95 (d, *J* = 12.0 Hz, 1H), 2.34-0.87 (m, 39H, PCy₃).

### Example 21

### Synthesis of Ru complex 4x

The synthetic procedure for preparation of ligand **3x** is the same as in Example 1 in 5.0 mmol scale. 0.96g of yellow oil product **3x** was obtained (yield: 76%).

Ligand **3x** ¹H-NMR (400 MHz, CDCl₃): δ 7.27 (dd, *J* = 4.5 Hz, 6.15 Hz, 1H), 7.21-7.17 (m, 1H), 6.95-6.88 (m, 2H), 6.82-6.75 (m, 2H), 6.64-6.60 (m, 1H), 6.55 (d, *J* = 5.7 Hz, 1H), 5.66 (d, *J* = 12.9 Hz, 1H), 5.32 (d, *J* = 8.1 Hz, 1H), 4.48 (m, 1H), 4.26 (s, 2H), 1.27 (d, *J* = 4.5 Hz, 6H).

The procedure for preparation of Ru complex **4x** is the same as in Example 2 in 1.0 mmol scale. 420 mg of green solid product **4x** was obtained (yield: 58%).

Ru complex **(4x)** ¹HNMR (400 MHz, CDCl₃): δ 19.55 (d, *J* = 9.9 Hz, Ru=CH), 8.14 (d, *J* = 8.1 Hz, 1H), 7.36-6.83 (m, 6H), 5.46 (d, *J* = 12.0 Hz, 1H), 5.13 (t, *J* = 6.0 Hz, 1H), 4.56 (m, 1H), 3.90 (d, *J* = 12.0 Hz, 1H), 2.30-1.25 (m, 39H, PCy₃).

### Example 22

### Synthesis of Ru complex 4y

The synthetic procedure for preparation of ligand **3y** is the same as in Example 1 in 5.0 mmol scale. 0.58g of yellow oil product **3y** was obtained (yield: 47%).

Ligand **3y** ¹H-NMR (400 MHz, CDCl₃): δ 7.33 (dd, *J* =5.6 Hz, 8.4Hz, 1H), 7.25 (dd, *J* = 2.8 Hz, 10 Hz, 1H), 7.05-6.82 (m, 3H), 6.81 (dd, *J* = 1.6 Hz, 8 Hz, 1H), 6.74-6.69 (m, 1H), 6.62 (dd, *J* = 1.6 Hz, 8Hz, 1H), 5.57 (d, *J* = 17.6 Hz, 1H), 5.40 (d, *J* = 11.2 Hz, 1H), 4.31 (s, 2H), 3.84 (s, 3H).

The procedure for preparation of Ru complex **4y** is the same as in Example 1 in 1.0 mmol scale. 267 mg of green solid product **4y** was obtained (yield: 37%).

Ru complex **(4y):** ¹HNMR (400 MHz, CDCl₃): δ 18.83 (s, Ru=CH), 7.50-6.39 (m, 11H), 5.21 (t, *J* = 12.4 Hz, 1H), 4.69-3.46 (m, 9H), 2.62-2.08 (m, 18H).

### Example 23

### Synthesis of Ru complex 4z

The structure of ligand **3z** is the same as **3y** for preparation of Ru complex **4z,** just another Ru complex intermediate **1a** was used instead of Ru intermediate **1b.**

The procedure for preparation of Ru complex **4z** is the same as in Example 2 in 1.0 mmol scale. 362 mg of green solid product **4z** was obtained (yield: 52%).

Ru complex **(4z)** ¹HNMR (400 MHz, CDCl₃): δ 19.35 (d, *J* = 9.9 Hz, Ru=CH), 8.11 (d, *J* = 8.1 Hz, 1H), 7.34-6.85 (m, 6H), 5.48 (d, *J* = 12.0 Hz, 1H), 5.27 (t, *J* = 6 Hz, 1H), 3.93 (d, *J* = 12.0 Hz, 1H), 3.88 (s, 3H), 2.33-1.24 (m, 33H, PCy₃).

### Example 24

### Synthesis of Ru complex 4aa

The structure of ligand **3aa** is the same as **3x** for preparation of Ru complex **4aa,** just another Ru complex intermediate **1b** was used instead of Ru intermediate **1a.**

The procedure for preparation of Ru complex **4aa** is the same as in Example 1 in 1.0 mmol scale. 631 mg of green solid product **4aa** was obtained (yield: 84%).

Ru complex (**4aa**) ¹HNMR (400 MHz, CDCl₃): δ 18.89 (s, Ru=CH), 7.60-6.45 (m, 11H), 5.13-3.52 (m, 8H), 2.95-2.10 (m, 18H), 0.95 (d, *J* = 6.4 Hz, 6H)

### Example 25

### Synthesis of Ru complex 4ab

The synthetic procedure for preparation of ligand **3ab** is the same as in Example 1 in 5.0 mmol scale. 0.32g of yellow oil product **3ab** was obtained (yield: 26%).

Ligand **3ab** ¹H-NMR (400 MHz, CDCl₃): δ 8.36 (d, *J* = 2.8 Hz, 1H), 8.05 (dd, *J* = 2.8 Hz, 8.4 Hz, 1H), 7.54 (d, *J* = 8.4 Hz, 1H), 7.01 (dd, *J* = 10.5 Hz, 17.1 Hz, 1H), 6.85-6.69 (m, 3H), 6.44 (d, *J* = 7.5 Hz, 1H), 5.86 (dd, *J* = 0.9 Hz, 17.1 Hz, 1H,), 5.53 (dd, *J* = 0.9Hz, 10.5 Hz, 1H), 4.46 (s, 2H), 3.87 (s, 3H)

The procedure for preparation of Ru complex **4ab** is the same as in Example 1 in 1.0 mmol scale. 300 mg of green solid product **4ab** was obtained (yield: 40%).

Ru complex (**4ab**) ¹HNMR (400 MHz, CDCl₃): δ 16.52 (s, Ru=CH), 7.58 (m, 1H), 7.09 (s, 4H), 6.93-6.60 (m, 6H), 4.52 (m, 1H), 4.35 (s, 2H), 4.18 (s, 4H, NCH₂CH₂N), 3.89 (s, 6H), 2.49 (s, 12H), 2.40 (s, 6H).

### Example 26

### Synthesis of Ru complex 4ac

The synthetic procedure for preparation of ligand **3ac** is the same as in Example 1 in 5.0 mmol scale. 1.09g of yellow oil product **3ac** was obtained (yield: 91%).

Ligand **3ac** ¹H-NMR (400 MHz, CDCl₃): δ 7.49 (s, 1H, NH), 7.27 (d, *J* = 7.5 Hz, 1H, aromatic H), 7.09-7.00 (m, 2H, aromatic H, CH=CH₂), 6.88-6.63 (m, 5H, aromatic H), 5.75 (d, *J* = 17.4 Hz, 1H, CH=CH₂), 5.38 (d, *J* = 10.8 Hz, 1H, CH=CH₂), 4.28 (s, 2H, NCH₂), 3.81 (s, 6H, OCH₃).

The procedure for preparation of Ru complex **4ac** is the same as in Example 1 in 1.0 mmol scale. 367 mg of green solid product **4ac** was obtained (yield: 50%).

Ru complex **(4ac)** ¹HNMR (400 MHz, CDCl₃): δ 19.03 (s, Ru=CH), 8.38 (d, *J* = 2.0 Hz, 1H), 7.69 (d, *J* = 16.0 Hz, 1H), 7.44 (d, *J* = 7.6 Hz, 1H), 7.21-7.03 (m, 5H), 6.83-6.59 (m, 3H), 5.24 (t, *J* =12.0 Hz, 1H), 4.66 (d, *J* =12.0 Hz, 1H), 4.45 (m, 1H), 4.20-4.05 (m, 4H, NCH₂CH₂N), 3.62 (d, *J* =12.0 Hz, 1H), 2.69-2.03 (m, 18H), 1.18 (d, *J* = 5.6 Hz, 6H).

### Example 27

### Synthesis of Ru complex 4ad

The synthetic procedure for preparation of ligand **3ad** is the same as in Example 1 in 5.0 mmol scale. 1.01g of yellow oil product **3ad** was obtained (yield: 79%).

Ligand **3ad** ¹H-NMR (400 MHz, CDCl₃): δ 7.48 (d, *J* = 2.1 Hz, 1H, aromatic H), 7.27-7.24 (m, 1H, aromatic H), 7.04 (dd, *J* = 18Hz, 10.8 Hz, 1H, CH=CH₂), 6.85-6.79 (m, 3H, aromatic H), 6.67-6.61 (m, 2H, aromatic H), 5.74 (dd, *J* = 18 Hz, 1.2 Hz, 1H, CH=CH₂), 5.28 (dd, *J* = 10.8 Hz, 1.2 Hz, 1H, CH=CH₂), 4.59-4.53 (m, 2H, OCH, NH), 4.29 (s, 2H, NCH₂), 3.86 (s, 3H, OCH₃), 1.37 (d, *J* = 6.4 Hz, 6H, OCH(CH₃)₂).

The procedure for preparation of Ru complex **4ad** is the same as in Example 1 in 1.0 mmol scale. 374 mg of green solid product **4ad** was obtained (yield: 49%).

Ru complex **(4ad)** ¹HNMR (400 MHz, CDCl₃): δ 16.52 (s, Ru=CH), 7.59 (m, 1H), 7.09 (s, 4H), 6.92-6.84 (m, 4H), 6.75-6.66 (m, 2H), 4.59 (m, 1H), 4.35 (s, 2H), 4.18 (s, 4H, NCH₂CH₂N), 3.89 (s, 3H), 2.49 (s, 12H), 2.40 (s, 6H, 18H), 0.93 (m, 6H).

### Example 28

### Synthesis of Ru complex 4ae

The synthetic procedure for preparation of ligand **3ae** is the same as in Example 1 in 5.0 mmol scale. 0.32g of yellow oil product **3ae** was obtained (yield: 27%).

Ligand **3ae** ¹HNMR (400 MHz, CDCl₃): δ 8.36 (d, *J* = 2.4 Hz, 1H), 8.05 (dd, *J* = 2.4 Hz, 8.4 Hz, 1H), 7.54 (d, *J* = 8.4 Hz, 1H), 7.01 (dd, *J* = 10.8 Hz, 17.1 Hz, 1H), 6.84-6.75 (m, 2H), 6.71-6.65 (m, 2H), 6.42 (dd, *J* = 1.8 Hz, 7.8 Hz, 1H), 5.85 (dd, *J* = 0.9 Hz, 17.1 Hz, 1H), 5.53 (dd, *J* = 0.9 Hz, 10.8 Hz, 1H), 4.58 (m, 1H), 4.47 (s, 1H), 1.36 (d, *J* = 6.0 Hz, 6H).

The procedure for preparation of Ru complex **4ae** is the same as in Example 1 in 1.0 mmol scale. 389 mg of green solid product **4ae** was obtained (yield: 50%).

Ru complex **(4ae)** ¹HNMR (400 MHz, CDCl₃): δ 19.03 (s,1H, Ru=CH), 8.38 (d, *J* = 2.0 Hz, 1H), 7.69 (d, *J* = 16.0 Hz, 1H), 7.44 (d, *J* = 7.6 Hz, 1H), 7.21-7.03 (m, 5H), 6.83-6.59 (m, 3H), 5.24 (t, *J* =12.0 Hz, 1H), 4.66 (d, *J* =12.0 Hz, 1H), 4.45 (m, 1H), 4.20-4.05 (m, 4H, NCH₂CH₂N), 3.62 (d, *J* = 12.0 Hz, 1H), 2.69-2.03 (m, 18H), 1.18 (d, *J* = 5.6 Hz, 6H).

### Example 29

### Synthesis of Ru complex 4af

The synthetic procedure for preparation of ligand **3af** is the same as in Example 1 in 5.0 mmol scale. 0.76g of yellow oil product **3af** was obtained (yield: 65%).

Ligand **3af** ¹H-NMR (400 MHz, CDCl₃): δ 7.38-7.34 (m, 2H, aromatic H), 7.22-7.10 (m, 2H, aromatic H, CH=CH₂), 7.01-6.88 (m, 4H, aromatic H), 5.63 (d, *J* = 17.1 Hz, 1H, CH=CH₂), 5.29 (d, *J* = 10.8 Hz, 1H, CH=CH₂), 4.20 (s, 2H, NCH₂), 3.88 (s, 3H, OCH₃), 2.63 (s, 3H, NCH₃).

The procedure for preparation of Ru complex **4af** is the same as in Example 1 in 1.0 mmol scale. 111 mg of green solid product **4af** was obtained (yield: 15%).

Ru complex (**4af**) ¹HNMR (400 MHz, CDCl₃): δ 18.54 (s,1H, Ru=CH), 7.45 (d, *J* = 8.0 Hz, 1H), 7.24-7.19 (m, 4H), 7.06-6.96 (m, 6H), 6.14 (d, *J* = 13.2 Hz, 1H), 5.39 (d, *J* = 13.2 Hz, 1H), 4.07-3.77 (m, 7H), 3.52 (s, 3H), 2.65-2.30 (m, 18H).

### Example 30

### Synthesis of Ru complex 4ag

The synthetic procedure for preparation of ligand **3ag** is the same as in Example 1 in 5.0 mmol scale. 0.84g of yellow oil product **3ag** was obtained (yield: 76%).

Ligand **3ag** ¹H-NMR (400 MHz, CDCl₃): δ = 7.26 Hz): 7.32 (dd, *J* = 5.70 Hz, 8.40 Hz, 1H), 7.24 (dd, *J* = 9.9 Hz, 2.4 Hz, 1H), 7.03-6.90 (m, 2H), 6.54-6.39 (m, 3H), 5.71 (dd, *J* = 1.2 Hz, 17.4 Hz, 1H), 5.37 (dd, *J* = 1.2 Hz, 10.8 Hz, 1H), 4.25 (s, 2H), 4.07 (bs, 1H), 3.81 (s, 3H), 3.76 (s, 3H).

The procedure for preparation of Ru complex **4ag** is the same as in Example 1 in 1.0 mmol scale. 302 mg of green solid product **4ag** was obtained (40% yield).

Ru complex **(4ag)** ¹HNMR (400 MHz, CDCl₃): δ 18.83 (s,1H, Ru=CH), 7.36-6.14 (m, 10H), 5.12 (t, *J* = 12.4 Hz, 1H), 4.50-3.42 (m, 12H), 2.62-2.05 (m, 18H).

### Example 31

### Synthesis of Ru complex 4ah

The synthetic procedure for preparation of ligand **3ah** is the same as in Example 1 in 5.0 mmol scale. 0.46g of yellow oil product **3ah** was obtained (yield: 38%).

Ligand **3ah** ¹H-NMR (400 MHz, CDCl₃): δ 7.34-7.23 (m, 2H), 7.03-6.91 (m, 2H), 6.69(dd, *J* = 1.2 Hz, 8.10 Hz , 1H), 7.52-6.45 (m, 2H), 5.72 (d, *J* = 17.4 Hz, 1H), 5.38 (d, *J* = 11.4 Hz, 1H), 4.32 (bs, 1H), 4.28 (s, 2H), 2.27 (s, 3H).

The procedure for preparation of Ru complex **4ah** is the same as in Example 1 in 1.0 mmol scale. 376 mg of green solid product **4ah** was obtained (yield: 51%).

Ru complex **(4ah)** ¹HNMR (400 MHz, CDCl₃): δ 18.90 (s,1H, Ru=CH), 7.60-6.36 (m, 10H), 5.25 (t, *J* = 12.0 Hz, 1H), 4.78 (d, *J* = 12.0 Hz, 1H), 4.05 (s, 4H, NCH₂CH₂N), 3.53 (s, 3H), 3.43 (d, *J* = 12.0 Hz, 1H), 2.56-2.13 (m, 21H).

### Example 32

### Synthesis of Ru complex 4aj

The synthetic procedure for preparation of ligand **3aj** is the same as in Example 1 in 5.0 mmol scale. 1.22g of yellow oil product **3aj** was obtained (yield: 90%).

Ligand **3aj** ¹H-NMR (400 MHz, CDCl₃): δ 7.35-7.21 (m, 2H), 7.03-6.77 (m, 4H), 6.71-6.58 (m, 2H), 5.71 (d, *J* = 17.7 Hz, 1H), 5.38 (d, *J* = 11.1 Hz, 1H), 4.31 (s, 2H), 4.06 (q, *J* = 11.1 Hz, 2H), 1.40 (t, *J* = 11.1Hz, 3H).

The procedure for preparation of Ru complex **4aj** is the same as in Example 2 in 1.0 mmol scale. 390 mg of green solid product **4aj** was obtained (yield: 55%).

Ru complex (**4aj**) ¹HNMR (400 MHz, CDCl₃): δ19.45 (d, J = 9.6 Hz, Ru=CH), 8.18 (d, *J* = 7.6 Hz, 1H), 7.40-7.33 (m, 2H), 7.21-7.11 (m, 2H), 6.95-6.88 (m, 2H), 5.52 (m, 1H), 5.23 (m, 1H), 4.16-3.94 (m, 3H), 2.36-0.81 (m, 36H, PCy₃).

### Example 33

### Synthesis of Ru complex 4ak

The synthetic procedure for preparation of ligand **3ak** is the same as in Example 1 in 5.0 mmol scale. 0.65g of yellow oil product **3ak** was obtained (yield: 52%).

Ligand **3ak** ¹H-NMR (400 MHz, CDCl₃): δ 7.47 (dd, *J* = 6.0 Hz, 8.4 Hz, 1H), 7.21 (dd, *J* = 10.4 Hz, 2.4 Hz, 1H), 7.13 (dd, *J* = 11.2 Hz, 17.2 Hz, 1H), 6.97-6.92 (m, 3H), 6.79 (d, *J* = 8.4 Hz, 1H), 5.63 (d, *J* = 17.2 Hz, 1H), 5.28 (d, *J* = 11.2 Hz,1H), 4.57 (m, 1H), 4.21 (s, 2H), 2.66 (s, 3H), 1.29-1.27 (m, 15H).

The procedure for preparation of Ru complex **4ak** is the same as in Example 1 in 1.0 mmol scale. 299 mg of green solid product **4ak** was obtained (yield: 37%).

Ru complex **(4ak)** ¹HNMR (400 MHz, CDCl₃): δ 19.08 (s,1H, Ru=CH), 7.97-6.33 (m, 10H), 5.08 (m, 2H), 4.34 (m, 1H), 4.02 (s, 4H, NCH₂CH₂N), 3.41 (m, 1H), 2.53-2.31 (m, 18H), 1.29 (s, 9H), 0.89-0.87 (m, 6H).

### Example 34

### Synthesis of Ru complex 4am

The synthetic procedure for preparation of ligand **3am** is the same as in Example 1 in 5.0 mmol scale. 1.10g of yellow oil product **3am** was obtained (yield: 86%).

Ligand **3am** ¹H-NMR (400 MHz, CDCl₃): δ 7.32 (m, 1H), 7.26-7.21 (m, 1H), 7.00-6.93 (m, 2H), 6.52-6.42 (m, 3H), 5.71 (d, *J* = 17.4 Hz, 1H), 5.37 (d, *J* = 11.1 Hz, 1H), 4.50 (m, 1H), 4.38 (m, 1H), 4.26 (s, 2H), 1.31 (m, 12H).

The procedure for preparation of Ru complex **4am** is the same as in Example 1 in 1.0 mmol scale. 437 mg of green solid product **4am** was obtained (yield: 54%).

Ru complex **(4am)** ¹HNMR (400 MHz, CDCl₃): δ 18.85 (s,1H, Ru=CH), 7.26-6.07 (m, 10H), 5.04 (t, *J* = 13.2 Hz, 1H), 4.48 (m, 1H), 4.39-4.33 (m, 2H), 4.15-4.02 (m, 4H, NCH₂CH₂N), 3.65 (m, 1H), 2.66-2.05 (m, 18H), 1.55 (m, 6H), 1.38 (m, 6H).

### Example 35

### Synthesis of Ru complex 4an

The synthetic procedure for preparation of ligand **3an** is the same as in Example 1 in 5.0 mmol scale. 0.66g of yellow oil product **3an** was obtained (yield: 41%).

Ligand **3an** ¹H-NMR (400 MHz, CDCl₃): δ 7.34-7.22 (m, 2H), 7.02-6.93 (m, 2H), 6.70 (d, *J* = 7.8 Hz, 1H), 6.48-6.43 (m, 1H), 5.71 (d, *J* = 17.4 Hz, 1H), 5.37 (d, *J* = 10.8 Hz, 1H), 4.46 (m, 1H), 4.40 (bs, 1H), 4.28 (s, 2H), 2.25 (s, 3H), 1.30 (d, *J=* 6.0 Hz, 6H).

The procedure for preparation of Ru complex **4an** is the same as in Example 1 in 1.0 mmol scale. 359 mg of green solid product **4an** was obtained (yield: 46%).

Ru complex **(4an)** ¹HNMR (400 MHz, CDCl₃): δ 18.98 (s,1H, Ru=CH), 7.66-6.39 (m, 10H), 5.17 (t, *J* = 13.2 Hz, 1H), 4.71 (d, *J* = 13.2 Hz, 1H), 4.36 (m, 1H), 4.06 (brs, 4H, NCH₂CH₂N), 3.42 (d, *J* = 13.2 Hz, 1H), 2.63-2.09 (m, 21H), 1.09 (m, 6H).

### Example 36

### Synthesis of Ru complex 4ap

The synthetic procedure for preparation of ligand **3ap** is the same as in Example 1 in 5.0 mmol scale. 0.70g of yellow oil product **3ap** was obtained (yield: 57%).

Ligand **3ap** ¹H-NMR (400 MHz, CDCl₃): δ 7.33-7.25 (m, 2H), 7.04 (dd, *J* = 10.8 Hz, 17.2 Hz, 1H), 6.96-6.92 (m, 1H), 6.79 (d, *J* = 2.4 Hz, 1H), 6.54 (d, *J* = 2.4 Hz, 1H), 5.72 (d, *J* = 17.2 Hz, 1H), 5.37(d, *J* = 10.8 Hz, 1H), 4.55 (m, 1H), 4.23 (s, 2H), 3.99 (bs, 1H), 1.40 (s, 9H), 1.29 (s, 9H), 1.20 (d, *J* = 6.0 Hz, 6H).

The procedure for preparation of Ru complex **4ap** is the same as in Example 1 in 1.0 mmol scale. 380 mg of green solid product **4ap** was obtained (yield: 44%).

Ru complex **(4ap)** ¹HNMR (400 MHz, CDCl₃): δ 18.99 (s,1H, Ru=CH), 7.45-6.36 (m, 9H), 5.05 (m, 2H), 3.98-3.91 (m, 5H), 3.72 (d, *J* = 13.2 Hz, 1H), 2.48-2.34 (m, 19H), 1.45-0.95 (m, 21H).

### Example 37

### Synthesis of Ru complex 4aq

The synthetic procedure for preparation of ligand **3aq** is the same as in Example 1 in 5.0 mmol scale. 0.63g of yellow oil product **3aq** was obtained (yield: 52%).

Ligand **3aq** ¹H-NMR (400 MHz, CDCl₃): δ 7.54 (d, *J =* 2.0 Hz, 1H), 7.32 (d, *J* = 8.4 Hz, 1H), 7.23 (dd, *J* = 2.0 Hz, 8.4 Hz, 1H), 6.98 (dd, *J* = 11.2 Hz, 17.2 Hz, 1H), 6.89 (td, *J* = 1.6 Hz, 7.6 Hz, 1H), 6.83 (td, *J* = 1.6 Hz, 8.0 Hz, 1H), 6.73 (td, *J* = 1.6 Hz, 8.0Hz, 1H), 6.59 (dd, *J* =1.6 Hz, 7.6 Hz, 1H), 5.74 (dd, *J* = 0.80 Hz, 17.2 Hz, 1H), 5.40 (dd, *J* = 0.80 Hz, 11.2 Hz, 1H), 4.33 (s, 2H), 3.86 (s, 3H).

The procedure for preparation of Ru complex **4aq** is the same as in Example 1 in 1.0 mmol scale. 665 mg of green solid product **4aq** was obtained (yield: 90%).

Ru complex (**4aq**) ¹H-NMR (400 MHz, CDCl₃): δ 18.75 (s,1H, Ru=CH), 7.50-7.44 (m, 2H), 7.04-6.36 (m, 9H), 5.32-5.21 (m, 1H), 4.65 (d, *J* = 13.2 Hz, 1H), 4.16-4.04 (m, 4H, NCH₂CH₂N), 3.59 (s, 3H), 3.48 (d, *J* = 13.2 Hz, 1H), 2.62-2.32 (m, 18H).

### Example 38

### Synthesis of Ru complex 4ar

The synthetic procedure for preparation of ligand **3ar** is the same as in Example 1 in 5.0 mmol scale. 0.56g of yellow oil product **3ar** was obtained (yield: 44%).

Ligand **3ar** ¹H-NMR (400 MHz, CDCl₃): δ 7.52 (d, *J* = 2.0 Hz, 1H), 7.31 (d, *J* = 8.4 Hz, 1H), 7.22 (dd, *J* = 2.0 Hz, 8.4 Hz, 1H), 6.96 (dd, *J* = 11.2 Hz, 17.2 Hz, 1H), 6.86-6.81 (m, 2H), 6.68 (td, *J* = 1.2 Hz, 7.6Hz 1H,), 6.56 (dd, *J* = 1.6 Hz, 7.6 Hz, 1H), 5.73 (dd, *J* = 0.8 Hz, 17.2 Hz, 1H,), 5.39 (dd, *J* = 0.8 Hz, 11.2 Hz, 1H), 4.56 (m, 1H), 4.33 (s, 3H), 1.35 (d, *J* = 6.0 Hz, 6H).

The procedure for preparation of Ru complex **4ar** is the same as in Example 1 in 1.0 mmol scale. 499 mg of green solid product **4ar** was obtained (yield: 65%).

Ru complex (**4ar**) ¹HNMR (300 MHz, CDCl3): δ 18.82 (s,1H, Ru=CH), 7.47-7.43 (m, 2H), 7.01-6.56 (m, 9H), 5.12-5.09 (m, 1H), 4.56-4.45 (m, 2H), 4.40-4.15 (m, 4H, NCH₂CH₂N), 3.48-3.45 (m, 1H), 2.64-2.04 (m, 18H), 1.10 (d, *J* = 6.4 Hz, 6H).

### Example 39

### Synthesis of Ru complex 4as

The synthetic procedure for preparation of ligand **3as** is the same as in Example 1 in 5.0 mmol scale. 0.45g of yellow oil product **3as** was obtained (yield: 34%).

Ligand **3as** ¹H-NMR (400 MHz, CDCl₃): δ 8.20 (d, *J* = 1.5 Hz, 1H), 7.90 (dd, *J* = 1.5 Hz, 8.0 Hz, 1H), 7.45 (d, *J* = 8.0 Hz 1H,), 7.01 (dd, *J* = 11.5 Hz, 17.0 Hz, 1H), 6.83-6.80 (m, 2H), 6.67 (td, *J* = 2.0 Hz, 7.0 Hz, 1H), 6.52 (dd, *J* = 2.0 Hz, 7.5 Hz, 1H), 5.80 (d, *J* = 17.0 Hz, 1H), 5.42 (d, *J* = 11.5 Hz, 1H), 4.56 (m, 1H), 4.42 (s, 2H), 3.93 (s, 3H), 1.34 (d, *J* = 6.5 Hz, 6H).

The procedure for preparation of Ru complex **4as** is the same as in Example 1 in 1.0 mmol scale. 467 mg of green solid product **4as** was obtained (yield: 59%).

Ru complex (**4as**) ¹HNMR (400 MHz, CDCl₃): δ 18.82 (s,1H, Ru=CH), 8.15 (dd, *J* = 6.4, 1.2 Hz, 2H), 7.51 (d, *J* = 1.2 Hz, 1H), 7.44 (d, *J* = 1.2 Hz, 1H), 7.05-6.99 (m, 5H), 8.15 (d, *J* = 6.4Hz, 2H), 6.59-6.56 (m, 1H), 5.22 (m, 1H), 4.63 (m, 1H), 4.41(m,1H), 3.96 (m, 4H, NCH₂CH₂N), 3.55-3.52 (m, 1H), 2.66-2.33 (m, 18H), 1.14 (d, *J* = 6.4 Hz, 6H).

### Example 40

### Synthesis of Ru complex 4at

The synthetic procedure for preparation of ligand **3at** is the same as in Example 1 in 5.0 mmol scale. 0.53g of yellow oil product **3at** was obtained (yield: 33%).

Ligand **3at** ¹H-NMR (400 MHz, CDCl₃): δ 7.91 (s, 1H), 7.63 (d, *J* = 8.0 Hz, 1H), 7.56 (d, *J* = 8.0 Hz, 1H), 7.03 (dd, *J* = 11.2 Hz, 17.2 Hz, 1H), 6.88-6.82 (m, 2H), 6.74 (t, *J* = 8.0 Hz, 1H), 6.53 (d, *J* = 7.6 Hz, 1H), 5.81 (d, *J* = 17.2 Hz, 1H), 5.49 (d, *J* = 11.2 Hz, 1H), 4.44 (s, 2H), 3.88(s, 3H), 2.74 (s, 6H).

The procedure for preparation of Ru complex **4at** is the same as in Example 1 in 1.0 mmol scale. 341 mg of green solid product **4at** was obtained (yield: 42%).

Ru complex (**4at**) ¹HNMR (400 MHz, CDCl₃): δ 19.02 (s,1H, Ru=CH), 7.87 (dd, *J* = 8.0, 1.2 Hz, 1H), 7.44 (dd, *J* = 7.2, 1.2 Hz, 1H), 7.25-7.03 (m, 9H), 5.37-5.30 (m, 1H), 4.76-4.74 (m, 1H), 4.16-4.01 (m, 4H, NCH₂CH₂N), 3.58-3.54 (m, 4H), 2.75 (s, 6H), 2.73-1.98 (m, 18H).

### Example 41

### Synthesis of Ru complex 4au

The synthetic procedure for preparation of ligand **3au** is the same as in Example 1 in 5.0 mmol scale. 0.58g of yellow oil product **3au** was obtained (yield: 39%).

Ligand **3au** ¹H-NMR (400 MHz, CDCl₃): δ 7.93 (s, 1H), 7.64 (d, *J* = 8.0 Hz, 1H), 7.50 (d, *J* = 8.0 Hz, 1H), 7.01 (dd, *J* = 10.8 Hz, 16.8 Hz, 1H), 6.84-6.69 (m, 3H), 6.49 (d, *J* = 7.6 Hz, 1H), 5.74 (d, *J* = 16.8 Hz, 1H), 5.47 (d, *J* = 10.8 Hz, 1H), 4.59-4.53 (m, 1H), 4.43 (s, 2H), 3.14 (t, *J* = 8 Hz, 4H), 1.51 (m, 4H), 1.36 (d, *J* = 5.6 Hz, 6H), 1.33-1.27 (m, 4H), 0.90 (t, *J* = 7.2 Hz, 6H).

The procedure for preparation of Ru complex **4au** is the same as in Example 1 in 1.0 mmol scale. 471 mg of green solid product **4au** was obtained (yield: 51%).

Ru complex (**4au**) ¹HNMR (300 MHz, CDCl3): δ 19.06 (s,1H, Ru=CH), 7.87 (d, *J* = 7.6Hz, 1H), 7.42 (d, *J* = 7.6Hz, 1H), 7.29 (d, *J* = 12.0 Hz, 1H), 7.11-6.56 (m, 8H), 5.22-5.19 (m, 1H), 4.63-4.64 (m, 1H), 4.45-4.42 (m, 1H), 4.14-4.01 (m, 4H, NCH₂CH₂N), 3.56-3.53 (m, 1H), 3.12-3.07 (m, 4H), 2.67-2.36 (m, 18H), 1.99-1.00 (m, 24H).

### Example 42

### Synthesis of Ru complex 4av

The synthetic procedure for preparation of ligand **3av** is the same as in Example 1 in 5.0 mmol scale. 0.65g of white solid product **3av** was obtained (yield: 39%). Ligand **3av** ¹H-NMR (400 MHz, CDCl₃): δ = 7.26 Hz) : 7.894 (s, 1H), 7.624 (d,1H, *J* = 8 Hz), 7.553 (d, *J* = 8 Hz, 1H), 7.017 (dd, *J* = 10.8 Hz, 17.2 Hz, 1H), 6.844-6.789 (m, 2H), 6.711 (d, *J* = 8 Hz, 1H), 6.698 (t, *J* = 8 Hz, 1H), 5.800 (d, *J =* 17.2 Hz, 1H), 5.493 (d, *J* = 10.8 Hz, 1H), 4.584 (m, 1H), 4.453 (s, 2H), 2.737 (s, 6H), 1.365 (d, *J* = 6 Hz, 6H).

The procedure for preparation of Ru complex **4av** is the same as in Example 1 in 1.0 mmol scale. 622 mg of green solid product **4av** was obtained (yield: 74%).

Ru complex (**4av**) ¹HNMR (300 MHz, CDCl3): δ 19.06 (s,1H, Ru=CH), 7.87 (d, *J* = 7.6Hz, 1H), 7.42 (d, *J* = 7.6Hz, 1H), 7.11-6.56 (m, 9H), 5.27-5.20 (m, 1H), 4.64-4.61 (m, 1H), 4.46-4.44 (m, 1H), 4.14-4.01 (m, 4H, NCH₂CH₂N), 3.59-3.56 (m, 1H), 3.12-3.07 (m, 4H), 2.75 (s, 6H), 2.67-2.36 (m, 18H), 1.13 (d, *J* = 6.0Hz, 6H).

### Example 43

### Synthesis of Ru complex 4aw

The structure of ligand **3aw** is the same as **3av** for preparation of Ru complex **4aw,** just another Ru complex reagent **1a** was used instead of Ru reagent **1b**.

The procedure for preparation of Ru complex **4aw** is the same as in Example 2 in 1.0 mmol scale. 626 mg of green solid product **4aw** was obtained (yield: 77%).

Ru complex (**4aw**) ¹HNMR (400 MHz, CDCl₃): δ 19.56 (d, *J* = 9.6 Hz, Ru=CH), 8.21 (d, *J* = 8.0 Hz, 1H), 8.09 (d, *J* = 2.0 Hz, 1H), 8.10 (dd, *J* = 7.6, 2 Hz, 1H), 7.34-6.87 (m, 4H), 5.47-5.44 (m, 1H), 5.33-5.27 (m, 1H), 4.62-4.56 (m, 1H), 3.99-3.96 (m, 1H), 2.80 (s, 6H), 2.30-1.24 (m, 39H, PCy₃).

### Example 44

### Synthesis of Ru complex 4ax

The synthetic procedure for preparation of ligand **3ax** is the same as in Example 1 in 5.0 mmol scale. 0.77g of yellow product **3ax** was obtained (yield: 55%). The product **3t** is confirmed by LC-MS (M+H⁺): m/z calculated: 431.2, found: 431.2, and directly used for preparation of the Ru complex **4ax.**

The procedure for preparation of Ru complex **4ax** is the same as in Example 1 in 1.0 mmol scale. 421 mg of green solid product **4ax** was obtained (yield: 47%).

Ru complex (**4ax**) ¹HNMR (400 MHz, CDCl₃): δ 18.99 (s,1H, Ru=CH), 7.88 (dd, *J* = 8.0, 2.0 Hz, 1H), 7.44 (dd, *J* = 7.2, 1.2 Hz, 1H), 7.28-6.63 (m, 9H), 5.35-5.28 (m, 1H), 4.75-4.72 (m, 1H), 4.16-4.12 (m, 4H, NCH₂CH₂N), 3.61 (s, 3H), 3.56-3.52 (m, 4H), 3.10-3.06 (m, 4H), 2.63-2.05 (m, 18H), 1.37-0.98 (m, 14H).

### Example 45

### Synthesis of Ru complex 4ay

The synthetic procedure for preparation of ligand **3ay** is the same as in Example 1 in 5.0 mmol scale. 0.56g of yellow oil product **3ay** was obtained (yield: 31%).

Ligand **3ay** ¹H-NMR (400 MHz, CDCl₃): δ 8.02 (d, *J* = 1.6 Hz, 1H), 7.72 (dd, *J* = 1.6 Hz, 8.4 Hz, 1H), 7.51 (d, *J* = 8.4 Hz, 1H), 7.01 (dd, *J* = 10.8 Hz, 17.6 Hz, 1H), 6.84-6.80 (m, 2H), 6.70 (td, *J* = 1.2 Hz, 7.6 Hz, 1H), 6.48 (dd, *J* = 1.2 Hz, 8.0 Hz, 1H), 5.80 (d, *J* = 17.6 Hz, 1H), 5.48 (d, *J* = 10.8 Hz, 1H), 4.67 (bs, 1H), 4.58 (m, 1H), 4.44 (s, 2H), 3.22-3.15 (bm, 1H), 1.81-1.77 (bm, 2H), 1.68-1.63 (bm, 2H), 1.36 (d, *J* = 6 Hz, 6H), 1.32-1.12 (m, 6H).

The procedure for preparation of Ru complex **4ay** is the same as in Example 1 in 1.0 mmol scale. 241 mg of green solid product **4ay** was obtained (yield: 27%).

Ru complex (**4ay**) ¹HNMR (400 MHz, CDCl₃): δ 19.03 (s, 1H, Ru=CH), 7.60 (d, *J* = 7.6 Hz, 1H), 7.43 (d, *J* = 3.6 Hz, 1H), 7.14 (s, 1H), 7.09-7.00 (m, 5H), 6.81-6.57(m, 3H), 5.22 (m, 1H), 4.64-4.61 (m, 1H), 4.64-4.42 (m, 2H), 4.15-4.02 (m, 4H, NCH₂CH₂N), 3.16 (m, 1H), 3.17 (m, 1H), 2.67-2.00 (m, 18H), 1.85-1.00 (m, 16H).

### Example 46

### Synthesis of Ru complex 4ba

The synthetic procedure for preparation of ligand **3ba** is the same as in Example 1 in 5.0 mmol scale. 0.96g of yellow oil product **3ba** was obtained (yield: 67%).

Ligand **3ba** ¹H NMR (CDCl₃, 400 MHz): δ 7.23 (m, 4H), 6.92 (m, 2H), 6.80 (m, 1H), 6.67 (m, 2H), 5.68 (d, 1H), 5.39 (d, 1H), 4.64 (s, 2H), 4.06 (s, 2H), 3.75 (s, 3H).

The procedure for preparation of Ru complex **4ba** is the same as in Example 1 in 1.0 mmol scale. 176 mg of green solid product **4ba** was obtained (yield: 22%).

Ru complex (**4ba**) ¹HNMR (400 MHz, CDCl₃): δ 18.74 (s, 1H, Ru=CH), 7.25-7.24 (m, 1H), 7.19(s, 1H), 7.14-7.04 (m, 7H), 6.93 (s, 1H), 6.71 (s, 1H), 6.41-6.40 (d, *J*=9.0 Hz, 1H), 6.10-6.07 (d, *J*=12.0 Hz, 1H), 4.52-4.49 (d, *J*=13.5Hz, 1H), 4.33-4.29 (d, *J*=18.5 Hz, 1H), 4.09 (s, 2H), 3.92 (s, 2H), 3.31 (s, 3H), 2.96-2.92 (d, *J*=19.0Hz, 1H), 2.83 (s, 3H), 2.71(s, 3H), 2.47 (s, 3H), 2.39 (s, 3H), 2.06 (s, 3H), 2.02 (s, 3H).

### Example 47

### Synthesis of Ru complex 4bb

The synthetic procedure for preparation of ligand **3bb** is the same as in Example 1 in 5.0 mmol scale. 1.13g of yellow oil product **3bb** was obtained (yield: 71%).

Ligand **3bb** ¹H NMR (CDCl₃, 400 MHz): δ 7.21 (m, 4H), 6.90 (m, 2H), 6.78 (m, 1H), 6.67 (d, 2H), 5.68 (d, 1H), 5.38 (d, 1H), 5.06 (m, 1H), 4.64 (s, 2H), 3.99 (s, 2H), 1.23 (d, 6H).

The procedure for preparation of Ru complex **4bb** is the same as in Example 1 in 1.0 mmol scale. 237 mg of green solid product **4bb** was obtained (yield: 30%).

Ru complex (**4bb**) ¹HNMR (400 MHz, CDCl₃): δ 18.74 (s, 1H, Ru=CH), 7.27-7.25 (dd, *J*=8.0, 3.0 Hz, 1H), 7.19 (s, 1H), 7.14-7.05 (m, 7H), 6.93 (s, 1H), 6.71 (s, 1H), 6.42-6.40 (d, *J*=9.0 Hz, 1H), 6.07-6.05 (d, *J*=12.5 Hz, 1H), 4.65-4.61 (m, 1H), 4.51-4.49 (d, *J*=12.5 Hz, 1H), 4.24-4.20 (d, *J*=18.0 Hz, 1H), 4.10 (s, 2H), 3.92 (s, 2H), 2.90-2.86 (d, *J*=18 Hz, 1H), 2.83 (s, 3H), 2.71 (s, 3H), 2.47 (s, 3H), 2.39 (s, 3H), 2.07 (s, 3H), 2.03 (s, 3H), 0.90-0.82 (d, *J*=33.0, 6.5 Hz, 6H).

### Example 48

### Synthesis of Ru complex 4bc

The synthetic procedure for preparation of ligand **3bc** is the same as in Example 1 in 5.0 mmol scale. 0.74g of yellow oil product **3bc** was obtained (yield: 43%).

Ligand **3bc** ¹H NMR (CDCl₃, 400 MHz): δ 7.23 (m, 2H), 6.92 (m, 2H), 6.81 (m, 2H), 6.67 (m, 2H), 5.67 (d, 1H), 5.37 (d, 1H), 5.05 (m, 1H), 4.57 (s, 2H), 3.98 (s, 2H), 3.77 (s, 3H), 1.22 (d, 6H).

The procedure for preparation of Ru complex **4bc** is the same as in Example 1 in 1.0 mmol scale. 578 mg of green solid product **4bc** was obtained (yield: 73%).

Ru complex (**4bc**) ¹HNMR (400 MHz, CDCl₃): δ 18.72 (s, 1H, Ru=CH), 7.24-7.22 (dd, *J*=8.5, 2.5 Hz, 1H), 7.16 (s, 1H), 7.07-7.04 (m, 4H), 6.91 (s, 1H), 6.75 (s, 1H), 6.66 (s, 1H), 6.64(s, 1H), 6.39-6.38 (d, *J*=8.0 Hz, 1H), 6.02-6.00 (d, *J*=12.0 Hz, 1H), 4.64-4.59 (m, 1H), 4.50-4.47 (d, *J*=13.0 Hz, 1H), 4.13-4.09 (d, *J*=18 Hz, 1H), 4.08 (s, 2H), 3.90 (s, 2H), 3.83(s, 3H), 2.81 (s, 3H), 2.81-2.79 (d, *J*=11.5 Hz, 1H), 2.69 (s, 3H), 2.45 (s, 3H), 2.39 (s, 3H), 2.08 (s, 3H), 2.01 (s, 3H), 0.89-0.81 (dd, J=34.0, 6.0 Hz, 6H).

### Example 49

### Synthesis of Ru complex 4bd

The synthetic procedure for preparation of ligand **3bd** is the same as in Example 1 in 5.0 mmol scale. 0.96g of yellow oil product **3bd** was obtained (yield: 52%).

Ligand **3bd** ¹H NMR (CDCl₃, 400 MHz): δ 7.21 (m, 2H), 7.16 (m, 2H), 6.86 (m, 2H), 6.58 (m, 2H), 5.68 (d, 1H), 5.39 (d, 1H), 5.06 (m, 1H), 4.60 (s, 2H), 3.97 (s, 2H), 1.23 (d, 6H).

The procedure for preparation of Ru complex **4bd** is the same as in Example 1 in 1.0 mmol scale. 236 mg of green solid product **4bd** was obtained (yield: 29%).

Ru complex (**4bd**) ¹HNMR (400 MHz, CDCl₃): δ 18.72 (s, 1H, Ru=CH), 7.28-7.26 (m, 1H), 7.19 (s, 1H), 7.10-7.05 (m, 6H), 6.94 (s, 1H), 6.82 (s, 1H), 6.41-6.39 (d, *J*=9.5 Hz, 1H), 6.07-6.04 (d, *J*=12.0 Hz, 1H), 4.68-4.64 (m, 1H), 4.45-4.43 (d, *J*=12.5 Hz, 1H), 4.24-4.20 (d, *J*=18.0 Hz, 1H), 4.09 (s, 2H), 3.93(s, 2H), 2.91-2.87 (d, *J*=18.5 Hz, 1H), 2.81 (s, 3H), 2.70 (s, 3H), 2.47 (s, 6H), 2.10 (s, 3H), 2.03 (s, 3H), 0.93-0.87 (dd, *J*=24.0, 7.0 Hz, 6H).

### Example 50

### Synthesis of Ru complex 4be

The synthetic procedure for preparation of ligand **3be** is the same as in Example 1 in 5.0 mmol scale. 1.46g of yellow oil product **3be** was obtained (yield: 84%).

Ligand **3be** H NMR (CDCl₃, 400 MHz): δ 7.23 (m, 2H), 6.91 (m, 4H), 6.61 (m, 2H), 5.68 (d, 1H), 5.38 (d, 1H), 5.05 (m, 1H), 4.58 (s, 2H), 3.95 (s, 2H), 1.23 (d, 6H).

The procedure for preparation of Ru complex **4be** is the same as in Example 1 in 1.0 mmol scale. 396 mg of green solid product **4be** was obtained (yield: 49%).

Ru complex (**4be**) ¹HNMR (400 MHz, CDCl₃): δ 18.71 (s, 1H, Ru=CH), 7.29-7.25 (dd, *J*=8.5, 2.5 Hz, 1H), 7.19 (s, 1H), 7.13-7.06 (m, 4H), 6.94 (s, 1H), 6.82-6.77 (m, 3H), 6.42-6.39 (dd, *J*=9.5, 2.5 Hz, 1H), 6.08-6.05 (d, *J*=13.0 Hz, 1H), 4.66-4.64 (m, 1H), 4.47-4.45 (d, *J*=12.5 Hz, 1H), 4.21-4.18 (d, *J*=18 Hz, 1H), 4.10 (s, 2H), 3.93 (s, 2H), 3.89-3.86 (d, *J*=18 Hz, 1H), 2.83 (s, 3H), 2.70 (s, 3H), 2.48 (s, 3H), 2.42 (s, 3H), 2.11 (s, 3H), 2.02 (s, 3H), 0.92-0.85 (dd, *J*=26.5, 7.0 Hz, 3H).

### Example 51

### Synthesis of Ru complex 4bf

The synthetic procedure for preparation of ligand **3bf** is the same as in Example 1 in 5.0 mmol scale. 0.68g of yellow oil product **3bf** was obtained (yield: 51%).

Ligand **3bf** ¹H NMR (CDCl₃, 400 MHz): δ 7.26 (m, 1H), 7.22 (m, 2H), 6.92 (m, 1H), 5.68 (d, 1H), 5.37 (d, 1H), 5.09 (m, 1H), 3.74 (s, 2H), 3.26 (s, 2H), 2.30 (m, 3H), 1.26 (d, 6H).

The procedure for preparation of Ru complex **4bf** is the same as in Example 1 in 1.0 mmol scale. 76 mg of green solid product **4bf** was obtained (yield: 10%).

Ru complex **(4bf)** ¹HNMR (400 MHz, CDCl₃): δ 18.54 (s, 1H, Ru=CH), 7.16-6.87 (m, 7H), 6.15-6.13 (dd, *J*=10.0, 2.0 Hz, 1H), 5.44-5.41 (d, *J*=13.5 Hz, 1H), 4.76-4.71 (m, 1H), 4.37-4.34 (d, *J*=15.5 Hz, 1H), 3.96 (s, 4H, NCH₂CH₂N), 3.07-3.05 (d, *J*=13 Hz, 1H), 2.75-2.40 (m, 18H), 1.66 (s, 3H), 1.21-1.17 (dd, *J*=13.0, 6.5 Hz, 6H).

### Example 52

### Synthesis of Ru complex 4bg

The synthetic procedure for preparation of ligand **3bg** is the same as in Example 1 in 5.0 mmol scale. 0.83g of yellow oil product **3bg** was obtained (yield: 59%).

Ligand **3bg** ¹H NMR (CDCl₃, 400 MHz): δ 7. 24 (m, 2H), 6.93 (m, 2H), 6.70 (m, 1H), 6.30 (m, 2H), 5.71 (d, 1H), 5.40 (d, 1H), 4.58 (s, 1H), 4.44 (m, 1H), 4.29 (s, 2H), 1.28 (d, 6H).

The procedure for preparation of Ru complex **4bg** is the same as in Example 1 in 1.0 mmol scale. 302mg of green solid product **4bg** was obtained (yield: 39%).

Ru complex (**4bg**) ¹HNMR (400 MHz, CDCl₃): δ 18.91 (s, 1H, Ru=CH), 7.60-7.58 (dd, *J* = 9.5, 2.5 Hz, 1H), 7.24-7.20 (m, 1H), 7.13-7.05 (m, 3H), 6.94-6.92 (dd, *J* = 8.0, 6.0 Hz, 1H), 6.80 (brs, 1H), 6.74-6.70 (m, 1H), 6.64-6.61 (dd, *J* = 9.0, 5.0 Hz, 1H), 6.45-6.43 (dd, *J* = 10.5, 3.0 Hz, 1H), 5.20-5.15 (t, *J* = 13.5, 1H, NCH₂), 4.69-4.67 (d, *J* = 12.5 Hz, 1H, NCH₂), 4.38-4.33 (m, 1H, OCH(CH₃)₂), 4.12-4.08(m, 4H, NCH₂CH₂N), 3.47-3.45 (d, *J* = 12.5 Hz, 1H, NH), 2.65 (s, 6H), 2.56 (s, 6H), 2.26 (s, 3H), 2.09 (s, 3H), 1.14-1.12 (dd, *J* = 6.0, 4.0 Hz, 6H, OCH(CH₃)₂).

### Example 53

### Synthesis of Ru complex 4bh

The synthetic procedure for preparation of ligand **3bh** is the same as in Example 1 in 5.0 mmol scale. 0.94g of yellow oil product **3bh** was obtained (yield: 78%).

Ligand **3bh** ¹H NMR (CDCl₃, 400 MHz): δ 7.99 (s, 1H), 7.95-7.93 (m, 1H), 7.55-7.53 (d, 1H), 7.36-7.32 (m, 2H), 7.30-7.23 (m, 2H), 7.03-6.98 (m, 1H), 6.66-6.61 (m, 2H), 5.72-5.68 (m, 1H), 5.36-5.34 (m, 1H), 4.46-4.45 (d, 2H), 3.85 (s, 3H).

The procedure for preparation of Ru complex **4bh** is the same as in Example 1 in 1.0 mmol scale. 542mg of green solid product **4bh** was obtained (yield: 74%).

Ru complex (**4bh**) ¹HNMR (400 MHz, CDCl₃): δ 18.89 (s, 1H, Ru=CH), 7.91-7.89 (d, *J* = 8.0 Hz, 1H), 7.76-7.74 (dd, *J* = 8.0, 1.5 Hz, 1H), 7.51-7.48 (td, *J* = 8.5, 7.0, 1.5 Hz, 1H), 7.25-7.21(td, *J* = 13.5, 11.0, 2.0 Hz, 1H), 7.19-7.16 (t, *J* = 8.0 Hz, 1H), 7.12-7.09 (t, *J* = 7.5 Hz, 2H), 7.04-7.03 (d, *J* = 7.0 Hz, 1H), 7.00-6.88 (m, 3H), 6.78-6.76 (d, *J* = 7.0 Hz, 1H), 6.65 (brs, 1H, NH), 6.64-6.59 (t, *J* = 12.5 Hz, 1H, NCH₂), 4.08(brs, 2H, NCH₂CH₂N), 3.99 (brs, 2H, NCH₂CH₂N), 3.72-3.69 (dd, *J* = 13.5, 2.0 Hz, 1H, NCH₂), 3.67 (s, 3H, COOCH₃), 2.62-2.03 (m, 18H).

### Example 54

### Synthesis of Ru complex 4bj

The synthetic procedure for preparation of ligand **3bj** is the same as in Example 1 in 5.0 mmol scale. 0.99g of yellow oil product **3bj** was obtained (yield: 82%).

Ligand **3bj** ¹H NMR (CDCl₃, 400 MHz): δ 7.58-7.57 (d, 1H), 7.38-7.36 (d, 1H), 7.32-7.25 (m, 2H), 7.08-7.00 (m, 3H), 6.74-6.70 (m, 1H), 6.65-6.63 (d, 1H), 5.73-5.69 (m, 1H), 5.33-5.30 (m, 1H), 4.90 (s, 1H), 4.35 (s, 2H), 2.63 (s, 6H).

The procedure for preparation of Ru complex **4bj** is the same as in Example 1 in 1.0 mmol scale. 508mg of green solid product **4bj** was obtained (yield: 69%).

Ru complex (**4bj**) ¹HNMR (400 MHz, CDCl₃): δ 18.90 (s, 1H, Ru=CH), 7.63-7.61 (d, *J* = 7.5 Hz, 1H), 7.49-7.46 (t, *J* = 7.0 Hz, 1H), 7.19-7.16 (t, *J* = 8.0 Hz, 1H), 7.11-6.95 (m, 6H), 6.87-6.84 (t, *J* = 8.0 Hz, 1H), 6.80-6.79 (d, *J* = 7.5 Hz, 1H), 6.72 (brs, 1H), 6.68-6.65 (d, *J* = 11.5 Hz, 1H, NCH₂), 5.50-5.45 (t, *J* = 13.0 Hz, 1H, NCH₂), 4.15-3.96 (m ,4H, NCH₂CH₂N), 3.51-3.48 (d, *J* = 13.5 Hz, 1H, NH), 2.66-2.30 (m, 21H, aromatic CH₃, NCH₃), 2.05 (brs, 3H, NCH₃).

### Example 106

### Synthesis of Ru complex 4i

Starting material **4-SM** (44g, 100mmol) and anhydrous ethanol (250mL) were added into a 500 mL three-necked flask filled with inert gas (Ar), followed by adding NaOEt (400mmol, 4.0eq) was quickly added with agitation. The reaction mixture was heated to 60°C. After the reaction was completed in 0.5-1.0 hr, 120 mL of water was added into flask, and the aqueous layer was extracted with pentane (200 mL×3), and the combined organic layers were washed with brine (150 mL×2) solution, then dried over NaSO₄ and concentrated to obtain about 50 mL of crude carbine intermediate **4-1** directly for next step at 0-5°C.

RuCl₂(PPh₃)₃ (29g, 30mmol) was dissolved in 250 mL of anhydrous DCM in a 500 mL three-neck flask filled with inert gas (Ar), and the DCM solution was cooled to -70°C, then the previously prepared crude carbine intermediate **4-1** (50 mL) was added into the DCM solution at -70°C. After 10 min, the solution was heated to room temperature, and CuCl (100mmol) was added. After completed in 30 min, the reaction solution was filtered and purified by silica gel column chromatography (eluting solution: n-hexane:DCM = 2:1 to pure DCM). The product was concentrated and washed by anhydrous n-hexane. After dried by vacuum, the Ru complex intermediate **4-2** (19.3g) was obtained.

The intermediate **4-2** (10.0mmol) and tricyclohexylphosphine (PCy₃, 20mmol, 2.0eq.) were dissolved in DCM (30mL) in a 250 mL three-neck flask filled with inert gas (Ar), then stirred at 20°C for about 30 min. After completed, the crude product was purified by flash column to obtain dark-green solid. The solid product was washed with anhydrous methanol and n-hexane to obtain green solid product **4i** (crude yield: 60-70%). The product **4i** is not stable and difficult to analyze the structure by ¹HNMR. But the crud Ru complex **4i** can be used directly to prepare **4j** in next step.

### Example 107

### Synthesis of Ru complex 4j

The Ru complex **4i** (5.0mmol) and a ligand H₂IMes(H)(CCl₃) (**4-4**, 10.0mmol, 2.0eq.) were dissolved in anhydrous toluene (30mL) in a 100 mL two-necked flask filled with Ar gas. The reaction mixture was heated to 80°C for 1.5hr. After the reaction was completed, the solution was cooled and filtered, then purified by flash column to obtain dark-green product. The crude product was washed by methanol and pentane-DCM to offer 2.3g of stable green solid product **4j** (yield: 59%).

Ru complex **4j** is confirmed by ¹HNMR (400 MHz, CDCl₃): δ 18.88 (s,1H,

Ru=CH), 7.57-6.44 (m, 11H, aromatic H), 5.36 (t, *J* = 13.2 Hz, 1H, NH), 4.16-4.02 (m, 5H, NCH₂, NCH₂CH₂N), 4.01 (d, *J* = 13.2 Hz, 1H, NCH₂), 2.75-2.00 (m, 19H, CH(CH₃)₂, aromatic CH₃), 1.01-0.90 (m, 6H, CH(CH3)₂).

### Example 108

### Synthesis of Ru complex 11h

The Ru complex **4j** (0.2mmol) and 4-chlorin pyridine 4-chlorin pyridine ligand (**4-5**, 2.0mmol) were reacted directly to form another Ru complex **11h** in 10 mL of anhydrous DCM in a 100 mL three-neck flask filled with inert gas (Ar). Preparation method and result of the Ru complex **11h** was the same as described in Example **92.** 619 mg of yellow-green solid product **11h** was obtained (yield: 73%).

Ru complex **11h** is confirmed by ¹HNMR (400 MHz, CDCl₃): δ 18.67 (s, 1H), 8.43 (s, 1H), 7.45-7.35 (m, 3H), 7.19-6.93 (m, 10H), 6.60 (d, *J* = 7.6 Hz, 1H), 4.15 (m, 6H), 2.52-2.28 (m, 19H), 1.08-0.89 (m, 6H).

### Example 110

### Synthesis of Ru complex 4x

**2j** (0.71g, 1.0 mmol) and a phosphine ligand PCy₃ (**4-3**, 1.5 mmol) were dissolved in 10 mL of anhydrous DCM in a 50mL three-neck flask filled with inert gas (Ar) and reacted to form the Ru complex **4x.** The reaction mixture was stirred until completed (monitored by TLC), the reaction product was precipitated in MeOH and filtered and purified by flask column. 0.56g of green solid product **4x** was obtained, yield: 78%.

The Ru complex **4x** prepared in this Example 110 is confirmed by ¹HNMR as the same as in Example 21.

### Example 111

### Synthesis of Ru complex 4aa

Ru complex **4x** (0.72g, 1.0 mmol) and heterocyclic ligand H₂IMes(H)(CCl₃) (**4-4**, 48g, 50 mmol) were dissolved in 10 mL of anhydrous Toluene in a 50mL three-neck flask filled with inert gas (Ar) and reacted to form the Ru complex **4x.** The reaction mixture was stirred until complete (monitored by TLC), the reaction solution was filtered and purified by flask column. 0.55g of green solid product **4x** was obtained (yield: 73%).

The Ru complex **4aa** prepared in this Example 111 is confirmed by ¹HNMR as the same as in Example 24.

### Example 112

### RCM reaction

RCM test by selecting the Ru Complexes of Examples **1-108** as Catalyst **General Procedure** for RCM Catalyzed by Ru Complex in DCM: Olefin substrate (**15** or **17**, 50mg/each, respectivrly) was dissolved in 1.0 mL of freshly distilled DCM in a 15mL two-neck round-bottom flask under Ar at 20-25 °C, then Ru catalyst (2 mol% of Ru complex selected from Examples **1-103,** respectively) was added into the DCM solution. The kinetic data for conversion of RCM reactions in Equations 1-2 were determined by HPLC at 10 min., 30 min. 1.5 hr, 3.0 hr and until completed overnight. The RCM product (**16** and **18**, respectivrly) was determined and the conversion results of RCM reactions were listed in Tables 1-1, 1-2, 1-3, 1-4, 1-5, and 2 above, respectively.

The RCM product **16** is confirmed by ¹HNMR (400 MHz, CDCl₃): δ 7.72 (d, *J* = 8.2 Hz,, 1H), 7.32 (d, *J* = 8.0 Hz, 1H), 5.66 (d, *J* = 4.4 Hz, 1H), 4.11 (d, *J* = 4.4 Hz, 1H), 2.42 (s, 3H). *m*/*z* calculated: 222.1; found: 222.2.

The RCM product **18** is confirmed by ¹HNMR (400 MHz, CDCl₃): δ 7.78 (d, 2H, *J* = 8.21Hz), 7.31 (m, 7H), 6.01 (m, 1H), 4.47 (m, 2H), 4.30 (m, 2H), 2.41 (s, 3H). (M+H⁺): *m*/*z* calculated: 300.1, found: 300.2.

### Example 113

### Catalyst Screening for Cross Metathesis Reaction

CM test by selecting Ru Complexes of Examples **1-108** as Catalyst(s)

**General Procedure** for CM Catalyzed by Ru Complex in DCM: Olefin substrate (**19**, 200mg/each, respectivrly) was dissolved in 3.0 mL of freshly distilled DCM in a 15mL two-neck round-bottom flask under Ar at 20-25 °C, then Ru catalyst (0.1 mol% of Ru complex selected from Examples **1-103,** respectively) was added into the DCM solution. The CM reaction results are described in section of Equation 3 above.

### Example 114

### Catalyst Screening for ROMP reaction without solvent

ROMP test by selecting Ru Complexes of Examples **1-108** as Catalyst(s) **General Procedure** for ROMP Catalyzed by Ru Complex without solvent for some liquid olefin substrates: Olefin substrate (**21, 23** or **25**, 5mL/each, respectivrly) was added into a 25mL flat-bottom bottle under Ar at 40-50°C, then Ru catalyst (0.1 mol% of Ru complex selected from Examples **1-103,** respectively) was added with agitation. The kinetic data and ROMP results for products **22, 24** and **26** are described in each section of Equation 4-6 above, respectively.

### Example 115

### Catalyst Screening for ROMP reaction with solvent

ROMP test by selecting Ru Complexes of Examples **1-108** as Catalyst(s) **General Procedure** for ROMP Catalyzed by Ru Complex in solution: 0.5g of cyclo-olefin substrate (**21, 23, 25, 27, 29**, or **31,** respectivrly) was dissolved in 10 mL of freshly distilled DCM in a 25mL two-neck round-bottom flask under Ar at 20-25 °C, then Ru catalyst (0.1 mol% of Ru complex selected from Examples **1-103,** respectively) was added into the DCM solution. The ROMP results for products **22, 24, 26 , 28, 30** and **32** are described in each section of Equation 4-9 above, respectively.

### Example 116

Catalyst Screening for Depolymerization of nitrile butadiene rubber by Metathesis Metathesis Depolymerization test by selecting Ru Complexes from Examples **1-108** as Catalyst(s)

**General Procedure** for depolymerization Catalyzed by Ru Complex: 60g of nitrile butadiene rubber (NBR) was dissolved in 500 mL of anhydrous chlorobenzene in a 1.0L well-sealed steel reactor under Ar at 30 °C, then the Ru catalyst **(4ab,** 0.04 wt%, one of Ru complexes selected from Examples **1-108)** was added into chlorobenzene solution. The depolymerization by Ru catalyst was conducted overnight to produce lower molecular weight rubber as shown in Equation 10. The depolymerized butyl rubber product was precipitated in MeOH, and dried over 97% of yield. The final rubber product has a Mw of **2.78E+05, a Mn of 1.586E+5, and a Mooney viscosity of 60.3.**

### Example 117

Catalyst Screening for Metathesis and Hydrogenation reactions of nitrile butadiene rubber

Metathesis and Hydrogenation test by selecting Ru Complexes from Examples **1-108** as Catalyst(s)

**General Procedure** for Metathesis and Hydrogenation Catalyzed by Ru Complex in solution: 60g of nitrile butadiene rubber (NBR, Raw Material) substrate was dissolved in 500 mL of anhydrous chlorobenzene in a 1.0L steel well-sealed reactor under Ar, then Ru catalyst (**4aa**, 0.07 wt% of Ru complex selected from Examples **1-108,** respectively) was added into chlorobenzene solution, followed by adding hydrogen under high pressure 5MPa, and finally heated upto 130°C overnight. The hydrogenated nitrile butadiene rubber product (HNBR) by Ru catalyst was prepared with lower molecular weight and higher hydrognation degree as shown in Equation 11. The depolymerized and hydrogenated butyl rubber product was precipitated in MeOH, and dried over 98% of yield. The final product has a Mw of 1.60E+05, a Mn of 1.12E+05, an Iodine value of 12.6, and a hydrogenation degree of greater than 95%.

### Example 118

### Catalyst Screening for Hydrogenation and Metathesis reactions of nitrile butadiene rubber

### Metathesis and Hydrogenation test by selecting selecting Ru Complexes from Examples 1-108 as Catalyst(s)

**General Procedure** for Metathesis and Hydrogenation Catalyzed by Ru Complex in solution: 60g of nitrile butadiene rubber (NBR) substrate was dissolved in 500 mL of anhydrous chlorobenzene in a 1.0L steel well-sealed reactor under Ar, then hydrogen was added under high pressure 5MPa, followed by adding Ru catalyst (**4aa**, 0.1 wt% of Ru complex selected from Examples **1-108)** into chlorobenzene solution, then heated upto 130°C overnight. The hydrogenated nitrile butadiene rubber product (HNBR) by Ru catalyst was prepared with higher hydrognation degree and lower molecular weight as shown in Equation 12. The hydrogenated butyl rubber product was precipitated in MeOH, and dried over 98% of yield. The final product has a Mw of 1.80E+05, a Mn of 1.07E+05, an Iodine value of 3.1, and a hydrogenation degree of greater than 99%.

## Claims

1. A transition metal complex having the following structure **IIa**, 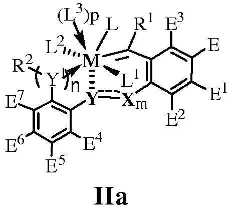 wherein:
m = 0 or 1, n = 1;
M is ruthenium;
L¹ and L² each is chloride anion;
L is an electron-donating ligand having the following structure **IIIa** or **IIId:** 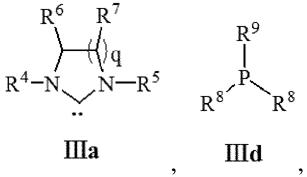
and in **IIIa**, q = 1, R⁴ and R⁵ each is 2,4,6-trimethylphenyl, R⁶ and R⁷ each is H; or in **IIId**, R⁸ and R⁹ each is cyclohexyl (Cy);
when m = 1, X is CH₂; Y is NH, or C₁-C₄ alkylamino; "**Y⁻⁻⁻X**" is single bond;
when m = 0, Y is NH, C₁-C₄ alkylamino,or C₆-C₉ arylamino;
n = 1, p = 0;
Y¹ is oxygen;
R¹ is H;
R² is methyl, ethyl, or isopropyl;
E is H, halogen, nitro, C₁-C₄ alkoxy, C₁-C₄ alkoxycarbonyl, C₁-C₈ alkylaminosulfonyl;
E¹ and E² are each H or halogen;
E³ is H;
E⁴ is H or C₁-C₄ alkyl;
E⁵ and E⁶ is H, halogen, C₁-C₄ alkyl or C₁-C₆ alkoxy;
E⁷ is H or C₁-C₄ alkyl.

2. The transition metal complex according to claim 1, wherein the transition metal complex is represented by any one of the following structures,

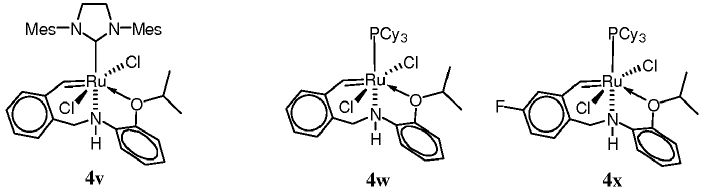

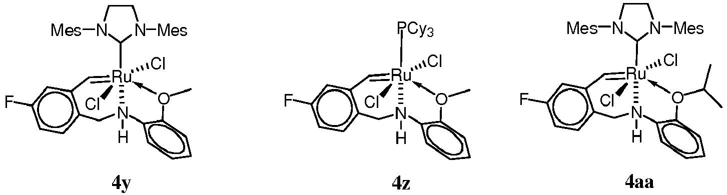

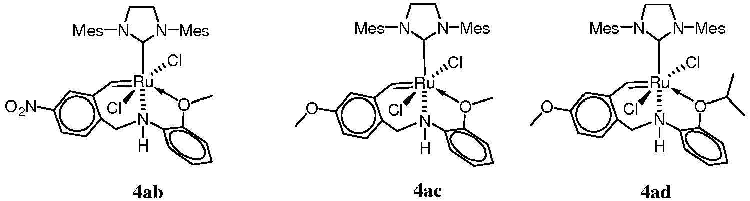

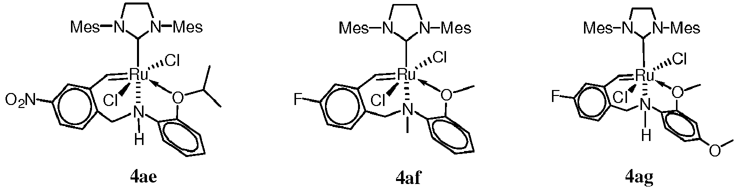

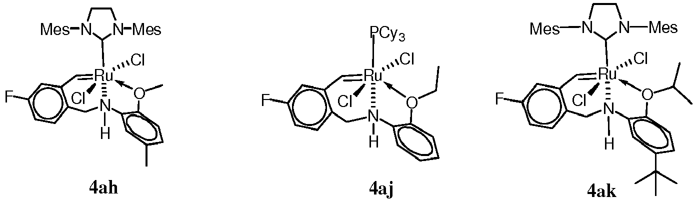

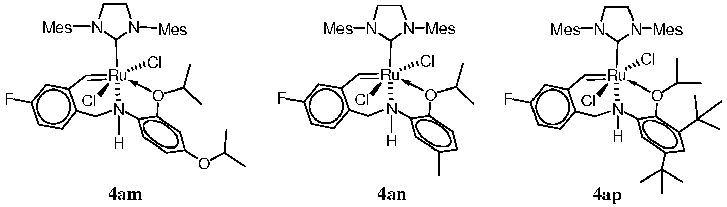

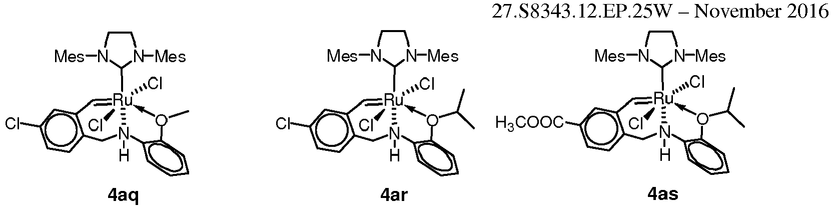

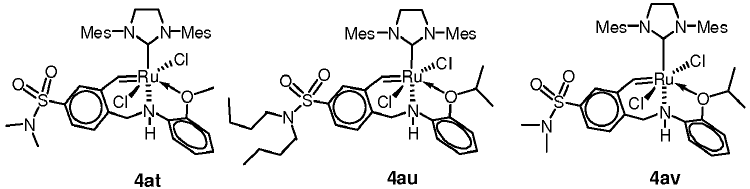

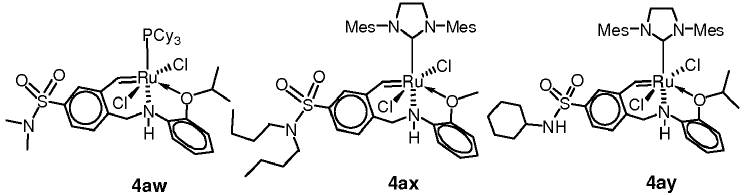

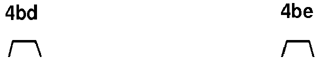

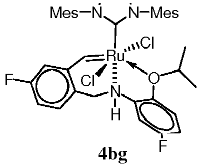

3. A method of carrying out a metathesis reaction with olefin substrate, comprising intramolecular ring-closing metathesis (RCM), intermolecular cross metathesis (CM), acyclic diene metathesis (ADMET) or ring-opening metathesis polymerization (ROMP) of cyclo-olefin substrate in the presence of one or more transition metal complexes of claim 1.

4. A method of making a modified nitrile butadiene rubber (NBR) or styrene-butadiene rubber (SBR) by depolymerization in the presence of one or more transition metal complexes of claim 1 at 30-100°C.

5. A method of making a depolymerized HNBR (hydrogenated nitrile butadiene rubber) or styrene-butadiene rubber (SBR) by adding one or more transition metal complexes of claim 1 first to carry out depolymerization of NBR, followed by adding hydrogen into the reaction under high pressure for hydrogenation at 60-150°C.

6. A method of making a hydrogenated nitrile butadiene rubber (HNBR) or styrene-butadiene rubber by adding hydrogen under high pressure first, followed by adding one or more transition metal complexes of claim 1 at 60-150°C.

7. A use of transition metal complexes of claim 1 in depolymerization of a rubber comprising at least one carbon-carbon double bond.

8. A use of transition metal complexes of claim 1 in hydrogenation of a rubber comprising at least one carbon-carbon double bond.

## Patentansprüche

1. Übergangsmetallkomplex mit der folgenden Struktur IIa, 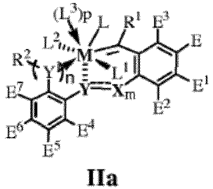 wobei
m = 0 oder 1, n = 1;
M Ruthenium ist;
L¹ und L² jeweils Chloridanionen sind;
L ein elektronenabgebender Ligand mit der folgenden Struktur IIIA oder IIId ist: 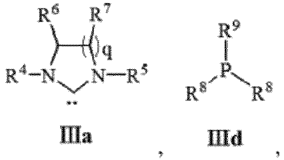
und in IIIA, q = 1, R⁴ und R⁵ jeweils 2,4,6-Trimethylphenyl sind, R⁶ und R⁷ jeweils H sind; oder in IIId sind R⁸ und R⁹ jeweils Cyclohexyl (Cy);
wenn m = 1, ist X CH₂; Y ist NH, oder C₁-C₄-Alkylamino; "**Y⁻⁻⁻X**" ist eine Einfachbindung;
wenn m = 0, ist Y NH, C₁-C₄-Alkylamino, oder C₆-C₉-Arylamino;
n = 1, p = 0;
Y¹ Sauerstoff ist;
R¹ H ist;
R² Methyl, Ethyl oder Isopropyl ist;
E H, Halogen, Nitro, C₁-C₄-Alkoxy, C₁-C₄-Alkoxycarbonyl, oder C₁-C₈-Alkylaminosulfonyl ist;
E¹ und E² jeweils H oder Halogen sind;
E³ H ist;
E⁴ H oder C₁-C₄ -Alkyl ist;
E⁵ und E⁶ H, Halogen, C₁-C₄-Alkyl oder C₁-C₆-Alkoxy sind;
E⁷ H oder C₁-C₄-Alkyl ist.

2. Übergangsmetallkomplex nach Anspruch 1, wobei der Übergangsmetallkomplex durch eine der folgenden Strukturen dargestellt wird,

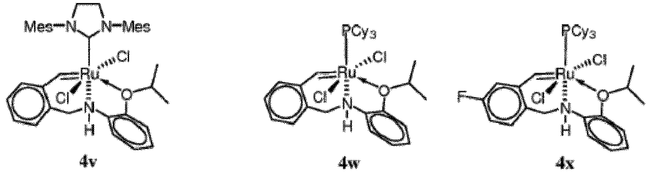

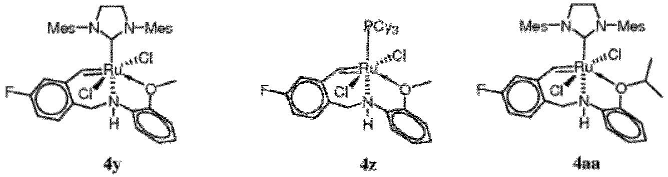

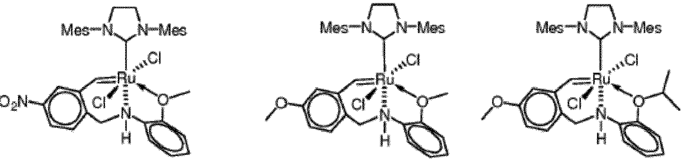

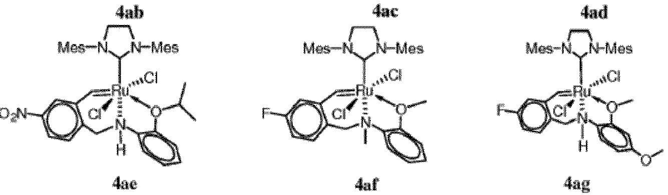

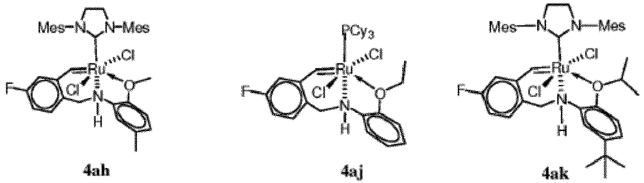

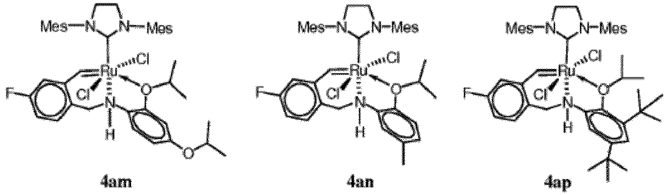

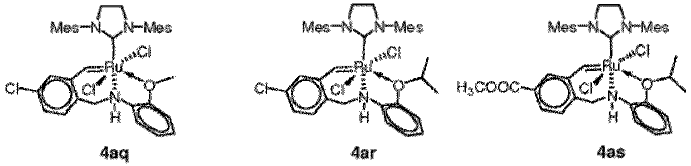

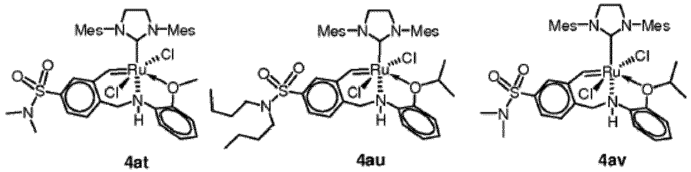

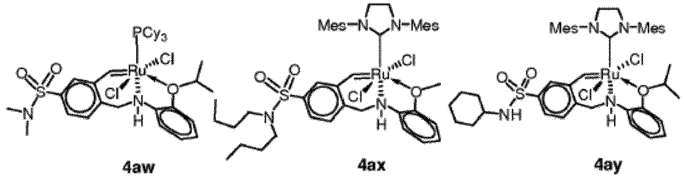

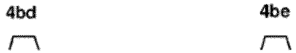

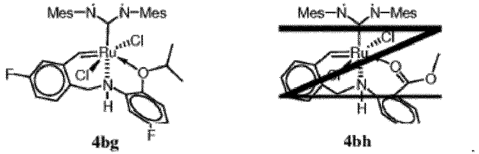

3. Verfahren zur Durchführung einer Metathesereaktion mit einem Olefinsubstrat, umfassend intramolekulare Ringschluß-Metathese (RCM), intermolekulare Kreuz-Metathese (CM), acyclische Dien-Metathese (ADMET) oder ringöffnende Metathesepolymerisation (ROMP) von Cycloolefin-Substrat in Gegenwart eines oder mehrerer Übergangsmetallkomplexe nach Anspruch 1.

4. Verfahren zur Herstellung eines modifizierten Nitrilbutadienkautschuks (NBR) oder Styrol-Butadien-Kautschuks (SBR) durch Depolymerisation in Gegenwart eines oder mehrerer Übergangsmetallkomplexe nach Anspruch 1 bei 30-100 °C.

5. Verfahren zur Herstellung eines depolymerisierten HNBR (hydrierter Nitril-Butadien-Kautschuk) oder StyrolButadien-Kautschuks (SBR) durch Zugabe eines oder mehrerer Übergangsmetallkomplexe nach Anspruch 1, um zunächst die Depolymerisation von NBR durchzuführen, gefolgt von der Zugabe von Wasserstoff in die Reaktion unter hohem Druck zur Hydrierung bei 60-150 °C.

6. Verfahren zur Herstellung eines hydrierten Nitrilbutadienkautschuks (HNBR) oder Styrol-Butadien-Kautschuks durch Zugabe von Wasserstoff unter hohem Druck, gefolgt von der Zugabe eines oder mehrerer Übergangsmetallkomplexe nach Anspruch 1 bei 60-150 °C.

7. Verwendung von Übergangsmetallkomplexen nach Anspruch 1 bei der Depolymerisation eines Kautschuks, der mindestens eine Kohlenstoff-Kohlenstoff-Doppelbindung umfasst.

8. Verwendung von Übergangsmetallkomplexen nach Anspruch 1 bei der Hydrierung eines Kautschuks, der mindestens eine Kohlenstoff-Kohlenstoff-Doppelbindung umfasst.

## Revendications

1. Complexe métallique de transition ayant la structure suivante IIa, 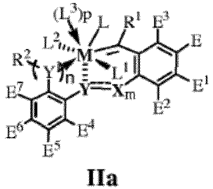 où
m = 0 ou 1, n = 1 ;
M est du ruthénium ;
L¹ et L² sont des anions chlorure ;
L est un ligand donneur d'électrons ayant la structure suivante IIIa ou IIId : 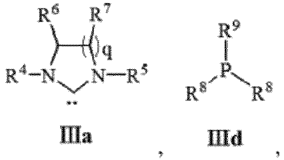
et dans IIIa, q = 1, R⁴ et R⁵ sont chacun du 2,4,6-triméthylphényle, R⁶ et R⁷ sont chacun de l'H ; ou dans IIId, R⁸ et R⁹ sont chacun du cyclohexyle (Cy) ; lorsque m = 1, X est du CH₂ ; Y est du NH, ou un alkylamino en C₁-C₄ ; "**Y⁻⁻⁻X**" est une liaison simple ;
lorsque m = 0, Y est du NH, de l'alkylamino en C₁-C₄ ou de l'arylamino en C₆-C₉ ;
n = 1, p = 0 ;
Y¹ est de l'oxygène ;
R¹ est de l'H ;
R² est du méthyle, de l'éthyle ou de l'isopropyle ;
E est de l'H, de l'halogène, de la nitro, de l'alkoxy en C₁-C₄, de l'alkoxycarbonyle en C₁-C₄, de l'alkylaminosulfonyle en C₁-C₈ ;
E¹ et E² sont tous deux de l'H ou de l'halogène ;
E³ est de l'H ;
E⁴ est de l'H ou de l'alkyle en C₁-C₄ ;
E⁵ et E⁶ sont de l'H, de l'halogène, de l'alkyle en C₁-C₄ ou de l'alkoxy en C₁-C₆ ;
E⁷ est de l'H ou de l'alkyle en C₁-C₄.

2. Complexe métallique de transition selon la revendication 1, dans lequel le complexe métallique de transition est représenté par l'une quelconque des structures suivantes,

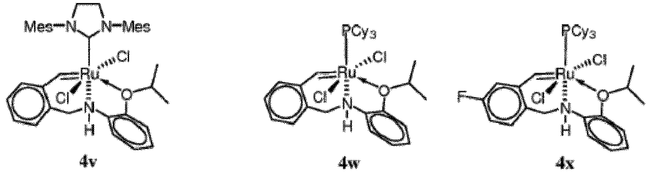

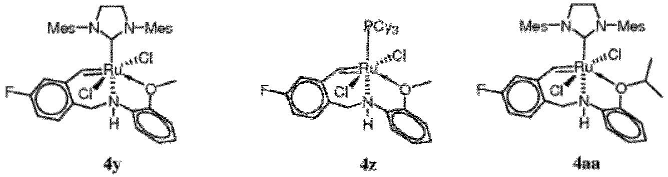

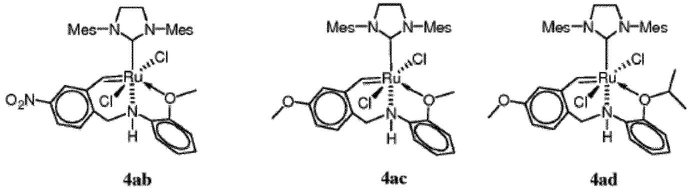

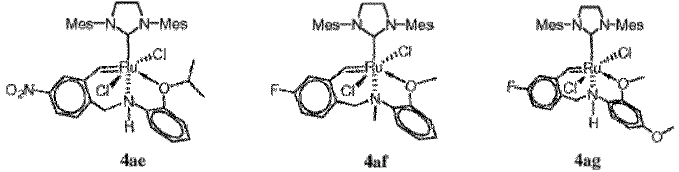

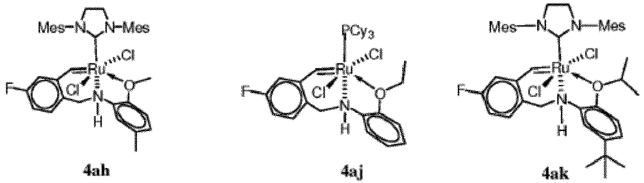

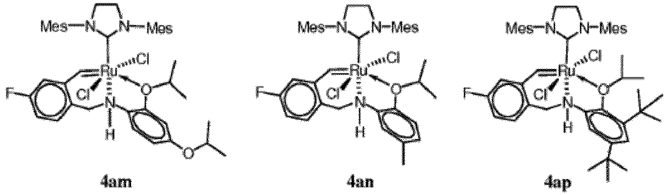

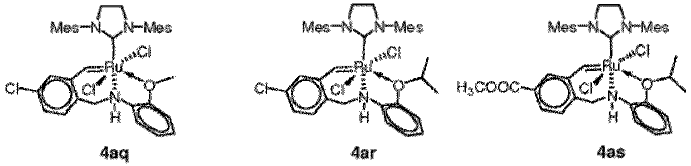

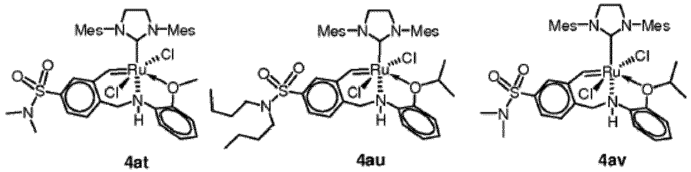

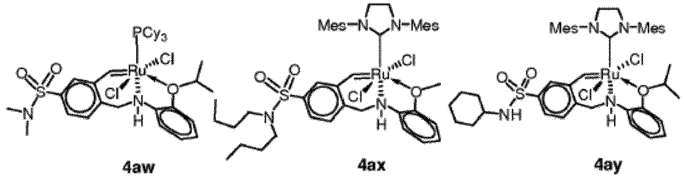

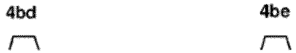

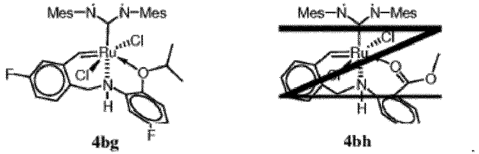

3. Procédé permettant d'effectuer une réaction de métathèse avec un substrat d'oléfine, comprenant une métathèse par fermeture de cycle intramoléculaire (RCM), une métathèse croisée intermoléculaire (CM), une métathèse de diène acyclique (ADMET) ou une polymérisation de métathèse par ouverture de cycle (ROMP) de substrat de cyclooléfine en présence d'un ou plusieurs complexes métalliques de transition selon la revendication 1.

4. Procédé de fabrication d'un caoutchouc nitrile modifié (NBR) ou d'un caoutchouc butadiène-styrène (SBR) par dépolymérisation en présence d'un ou plusieurs complexes métalliques de transition selon la revendication 1 à 30-100 °C.

5. Procédé de fabrication d'un HNBR dépolymérisé (caoutchouc nitrile hydrogéné) ou d'un caoutchouc butadiène-styrène (SBR) en ajoutant un ou plusieurs complexes métalliques de transition selon la revendication 1, d'abord pour effectuer une dépolymérisation du NBR, suivi d'un ajout d'hydrogène dans la réaction sous haute pression pour une hydrogénation à 60-150 °C.

6. Procédé de fabrication d'un caoutchouc nitrile hydrogéné (HNBR) ou d'un caoutchouc butadiène-styrène en ajoutant d'abord de l'hydrogène sous haute pression suivi par l'ajout d'un ou plusieurs complexes métalliques de transition selon la revendication 1 à 60-150 °C.

7. Utilisation de complexes métalliques de transition selon la revendication 1 dans la dépolymérisation d'un caoutchouc comprenant au moins une liaison double carbone-carbone.

8. Utilisation de complexes métalliques de transition selon la revendication 1 dans l'hydrogénation d'un caoutchouc comprenant au moins une liaison double carbone-carbone.
